# EUROPEAN PATENT APPLICATION

(11) **EP 1 391 209 A1**
(43) Date of publication of application: **25.02.2004**
(21) Application number: 02776487.7
(22) Date of filing: 17.05.2002
(51) Int. Cl.: A61K 38/00, A61K 39/395, A61K 47/12, A61K 47/20, A61K 47/22, A61K 9/08

(54) **PROTEIN FORMULATIONS**

(30) Priority: 30.05.2001 JP 2001162512
(71) Applicant: CHUGAI SEIYAKU KABUSHIKI KAISHA, Tokyo, 115-8543 (JP)
(72) Inventor: IMOTO, Taiji, Fukuoka-shi, Fukuoka 815-0042 (JP); UEDA, Tadashi, Munakata-gun, Fukuoka 811-3221 (JP); KAMEOKA, Daisuke, Kita-ku, Tokyo 115-8543 (JP)
(74) Representative: VOSSIUS & PARTNER
(86) International application number: PCT/JP2002/004793
(87) International publication number: WO 2002/098445

(57) **Abstract**

A protein formulation with improved stability, which comprises a physiologically active protein in one or more buffers selected from the group consisting of acetate buffer, imidazole buffer, a buffer of aminoethanesulfonate or a derivative thereof, and a buffer of aminopropanesulfonate or a derivative thereof.

## Description

### TECHNICAL FIELD

The present invention relates to protein formulations with improved stability. More specifically, the present invention relates to improving the stability of physiologically active proteins (e.g., antibodies) in a highly concentrated solution form by using a buffer such as acetate buffer, imidazole buffer, 2-morpholinoethanesulfonate buffer or 3-morpholinopropanesulfonate buffer.

### BACKGROUND ART

Advances in gene recombinant technology have enabled a pharmaceutical application of physiologically active proteins such as antibodies, enzymes, hormones and cytokines. To ensure a stable supply of high quality proteins, it is necessary to establish production and storage conditions capable of maintaining the structure and activity of the proteins.

When proteins are stored in a highly concentrated solution form, they are usually associated with a problem of deterioration including the formation of insoluble aggregates; and therefore, it is necessary to prevent the phenomena of deterioration.

For example, antibodies such as immunoglobulins, monoclonal antibodies and humanized antibodies are unstable proteins and more likely to undergo physical and chemical changes (e.g., association, aggregation) due to stresses applied during virus removal in the purification process, including filtration stress, concentration stress and heat stress.

To prevent protein deterioration for stable storage, stabilization by freeze-drying has been conventionally and widely used. However, proteins are likely to undergo denaturation or chemical changes due to mechanical stresses during freezing and subsequent drying; and hence a cryoprotective agent should be added to avoid these phenomena in such a strategy.

It has also been found that stabilization effects are obtained by adding a stabilizer for preventing chemical and physical changes, including high-molecular weight materials such as proteins (e.g., human serum albumin, purified gelatin) or low-molecular weight materials such as polyols, amino acids and surfactants. However, when added as stabilizers, organism-derived high-molecular weight materials like proteins are disadvantageous in requiring very complicated processes to remove contaminants, such as viruses, originating from the stabilizers per se. Also, when heat treatment is carried out for the purpose of virus inactivation, such stabilizers may cause problems such as association and aggregation due to heat stress.

Further, as to the formation of insoluble aggregates in a protein solution, it is caused by the formation of denatured proteins, i.e., denatured intermediates; there has been a need to develop a method for preventing the formation of insoluble aggregates.

The object of the present invention is to provide stable formulations containing physiologically active proteins (e..g., antibodies, enzymes, hormones and cytokines) by improving the stability of the physiologically active proteins in a highly concentrated solution form to prevent the formation of denatured intermediates and insoluble aggregates.

### DISCLOSURE OF THE INVENTION

As a result of extensive and intensive efforts made to achieve the above object, the inventors of the present invention have found that the stability of proteins can be increased in one or more buffers, particularly of about pH 5 to 7, selected from the group consisting of acetate buffer, imidazole buffer, a buffer of aminoethanesulfonate or a derivative thereof, and a buffer of aminopropanesulfonate or a derivative thereof, thereby preventing the formation of denatured intermediates as well as insoluble aggregates. This finding led to the completion of the present invention.

Namely, the present invention provides the following:
(1) a protein formulation with improved stability, which comprises a physiologically active protein in one or more buffers selected from the group consisting of acetate buffer, imidazole buffer, a buffer of aminoethanesulfonate or a derivative thereof, and a buffer of aminopropanesulfonate or a derivative thereof;
(2) the protein formulation of (1) above, wherein the buffer(s) has a pH of 5 to 7;
(3) the protein formulation of (1) or (2) above, wherein said aminoethanesulfonate or a derivative thereof is selected from the group consisting of:
   2-morpholinoethanesulfonate (MES),
   N-(2-acetamide)-2-aminoethanesulfonate (ACES),
   N,N-bis(2-hydroxyethyl)-2-amlnoethanesulfonate (BES),
   2-[4-(2-hydroxyethyl)-1-piperazinyl]ethanesulfonate (HEPES),
   piperazine-1,4-bis(2-ethanesulfonate) (PIPES), and
   N-tris(hydroxymethyl)methyl-2-aminoethanesulfonate (TES);
(4) the protein formulation of (1) or (2) above, wherein said aminopropanesulfonate or a derivative thereof is selected from the group consisting of:
   3-morpholinopropanesulfonate (MOPS),
   2-hydroxy-3-[4-(2-hydroxyethyl)-1-piperazinyl]propanesulfonate (HEPPSO),
   2-hydroxy-3-morpholinopropanesulfonate (MOPSO), and
   piperazine-1,4-bis(2-hydroxy-3-propanesulfonate) (POPSO);
(5) the protein formulation of (1) or (2) above, wherein the protein is selected from an antibody, an enzyme, a cytokine and a hormone;
(6) the protein formulation of (5) above, wherein the protein is an antibody;
(7) the protein formulation of (6) above, wherein the antibody is a monoclonal antibody;
(8) the protein formulation of (7) above, wherein the monoclonal antibody is a humanized antibody;
(9) a method for improving the stability of a protein formulation, which comprises dissolving a physiologically active protein in one or more buffers selected from the group consisting of acetate buffer, imidazole buffer, a buffer of aminoethanesulfonate or a derivative thereof, and a buffer of aminopropanesulfonate or a derivative thereof;
(10) a method for stabilizing a protein-containing sample under heat treatment, which comprises performing heat treatment on the protein-containing sample in such a form that a physiologically active protein is contained in one or more buffers selected from the group consisting of acetate buffer, imidazole buffer, a buffer of aminoethanesulfonate or a derivative thereof, and a buffer of aminopropanesulfonate or a derivative thereof; and
(11) a method for stabilizing the Fc region of an antibody, which comprises dissolving the antibody in one or more buffers selected from the group consisting of acetate buffer, imidazole buffer, a buffer of aminoethanesulfonate or a derivative thereof, and a buffer of aminopropanesulfonate or a derivative thereof.

### BRIEF DESCRIPTION OF DRAWINGS

Figure 1 shows the time course of fluorescence intensity (ANS binding) after heat treatment at 60°C.
Figure 2 shows the time course of native peak area after heat treatment at 60°C, as separated by cation-exchange HPLC.
Figure 3 shows the time course of denatured intermediate peak area after heat treatment at 60°C, as separated by cation-exchange HPLC.
Figure 4 shows the time course of monomer (native) peak area after heat treatment at 60°C, as separated by gel filtration HPLC.
Figure 5 shows the time course of soluble aggregate (denatured intermediate) peak area after heat treatment at 60°C, as separated by gel filtration HPLC.
Figure 6 shows the fluorescence intensity of ANS- ' binding measured for each sample after heat treatment at 60°C for 4 weeks.
Figure 7 shows the results of cation-exchange HPLC analysis performed on each sample after heat treatment at 60°C for 4 weeks: (A) % native peak, (B) % denatured intermediate peak and (C) % unknown peak.
Figure 8 shows the results of gel filtration HPLC analysis performed on each sample after heat treatment at 60°C for 4 weeks: (A) % monomer peak and (B) % soluble aggregate peak.
Figure 9 shows the % residual H chain calculated for each sample after heat treatment at 60°C for 4 weeks.
Figure 10 shows the ammonia content measured for each sample after heat treatment at 60°C for 4 weeks.
Figure 11 shows the isomerization degree of asparagine and aspartic acid residues measured for each sample after heat treatment at 60°C for 4 weeks. In the figure, Asx denotes the sum of asparagine residues and aspartic acid residues.
Figure 12 shows the time course of % residual uncleaved antibody H chain or peptide during heat treatment at 60°C.
Figure 13 shows the time course of % residual monomer peak in the heat-treated Fab fragment sample, as measured by gel filtration HPLC.
Figure 14 shows the time course of % residual monomer peak in the heat-treated Fc fragment sample, as measured by gel filtration HPLC.
Figure 15 shows the time course of fluorescence intensity at 470 nm in the heat-treated Fab fragment sample, as measured by ANS-binding fluorescence spectroscopy.
Figure 16 shows the time course of fluorescence intensity at 470 nm in the heat-treated Fc fragment sample, as measured by ANS-binding fluorescence spectroscopy.
Figure 17 shows the thermogram of each sample (Fab fragment, Fc fragment and the whole antibody), as measured by differential scanning calorimetry (DSC).
Figure 18 shows the assessment of reversibility in each denaturation step of the whole antibody.
Figure 19 shows the assessment of reversibility in denaturation of the Fab fragment.
Figure 20 shows the assessment of reversibility in each denaturation step of the Fc fragment.
Figure 21 shows the effect of DSC heating rate on the denaturation midpoint temperature in each denaturation step of the whole antibody.
Figure 22 shows the effect of DSC heating rate on the denaturation midpoint temperature in denaturation of the Fab fragment.

### BEST MODE FOR CARRYING OUT THE INVENTION

Examples of a physiologically active protein as used herein include, but are not limited to, antibodies, enzymes, cytokines and hormones. Specific examples include, but are not limited to, hematopoietic factors such as granulocyte colony-stimulating factor (G-CSF), granulocyte-macrophage colony-stimulating factor (GM-CSF), erythropoietin (EPO) and thrombopoietin, cytokines such as interferons, IL-1 and IL-6, immunoglobulins, monoclonal antibodies, humanized antibodies, tissue plasminogen activator (tPA), urokinase, serum albumin, blood coagulation factor VIII, leptin, insulin, and stem cell growth factor (SCF). Among these proteins, preferred are immunoglobulins, and more preferred are monoclonal antibodies and humanized antibodies.

A "physiologically active protein" refers to a protein having substantially the same biological activities as a corresponding physiologically active protein of mammalian (especially human) origin. Such a protein may be natural or recombinant, preferably recombinant. A recombinant protein encompasses those having the same amino acid sequence as the corresponding natural protein, as well as those comprising deletion, substitution or addition of one or more amino acid residues in the amino acid sequence, but retaining the biological activities as mentioned above. Further, the physiologically active protein as used herein includes those chemically modified with PEG, etc.

When a physiologically active protein is a monoclonal antibody, it may be prepared in any manner. In principle, a monoclonal antibody can be produced using known techniques by immunizing a sensitized antigen in a standard manner for immunization, fusing the resulting immunocytes with known parent cells in a standard manner for cell fusion, and then screening monoclonal antibody-producing cells in a standard manner for screening. Further, the monoclonal antibody as used herein is not limited to those produced by hybridomas, but includes chimeric antibodies which are artificially modified, e.g., for the purpose of reducing xenoantigenicity to human. Alternatively, reshaped humanized antibodies may also be used in the present invention. Such antibodies are prepared by replacing complementarity-determining regions of human antibodies with those of non-human mammalian (e.g., mouse) antibodies; standard recombinant procedures for this purpose are also known. Such known procedures may be used to give reshaped humanized antibodies.

If necessary, amino acid substitutions may be made in the framework (FR) regions of antibody variable domains such that the complementarity-determining regions of reshaped humanized antibodies form appropriate antigen-binding sites (Sato et al., Cancer Res. 53:1-6, 1993). A humanized anti-IL-6 receptor antibody (hPM-1) can be presented as a preferred example for such reshaped humanized antibodies (see WO92/19759). In addition to this, a humanized anti-HM1.24 antigen monoclonal antibody (see WO98/14580), a humanized anti-parathyroid hormone-related peptide antibody (anti-PTHrP antibody)(see WO98/13388), a humanized anti-tissue factor antibody (see WO99/51743) and the like are also preferred for use in the present invention.

Further, human antibodies produced in transgenic animals and the like are also preferred.

Furthermore, the antibody as used herein encompasses antibody fragments including Fab, (Fab')₂, Fc, Fc' and Fd, as well as reshaped antibodies including monovalent or polyvalent single chain antibodies (scFV).

As used herein, a "sample containing a physiologically active protein" or an "antibody-containing sample" refers to a sample containing any protein or antibody, regardless of whether derived from organisms or produced recombinantly. It preferably refers to a culture medium of mammalian cells (e.g., CHO cells) containing physiologically active protein or antibody molecules produced by culture, which may further be subjected to partial purification or other specific treatment.

The inventors of the present invention have studied various factors involved in heat treatment-induced denaturation of antibody-containing samples. As a result, they have found that when heat treatment at 80°C for 2 hours or at 60°C for 2 or 4 weeks is performed on antibody molecules dissolved in acetate buffer, imidazole buffer, a buffer of aminoethanesulfonate or a derivative thereof or a buffer of aminopropanesulfonate or a derivative thereof, these buffers achieve a larger stabilization effect than phosphate or citrate buffer because they allow reduction of denatured intermediates (i.e., soluble associated molecules), prevention of the denaturation phenomenon, as well as avoidance of insoluble aggregate formation. In particular, it has also been observed that these buffers inhibit further aggregation of denatured intermediates into insoluble aggregates.

The protein formulation of the present invention may be prepared by dissolving a physiologically active protein and various ingredients as shown below into one or more buffers selected from the group consisting of acetate buffer, imidazole buffer, a buffer of aminoethanesulfonate or a derivative thereof, and a buffer of aminopropanesulfonate or a derivative thereof. These buffers may be used alone or in combination.

Preferred examples of aminoethanesulfonate or a derivative thereof include, but are not limited to:
2-morpholinoethanesulfonate (MES),
N-(2-acetamide)-2-aminoethanesulfonate (ACES),
N,N-bis(2-hydroxyethyl)-2-aminoethanesulfonate (BES),
2-[4-(2-hydroxyethyl)-1-piperazinyl]ethanesulfonate (HEPES),
piperazine-1,4-bis(2-ethanesulfonate)(PIPES), and
N-tris(hydroxymethyl)methyl-2-aminoethanesulfonate (TES). Any derivative may be used as long as it has a pKa of about 5 to 8. The most preferred is 2-morpholinoethanesulfonate (MES).

Preferred examples of aminopropanesulfonate or a derivative thereof include, but are not limited to:
3-morpholinopropanesulfonate (MOPS),
2-hydroxy-3-[4-(2-hydroxyethyl)-1-piperazinyl]propanesulfonate (HEPPSO),
2-hydroxy-3-morpholinopropanesulfonate (MOPSO), and
piperazine-1,4-bis(2-hydroxy-3-propanesulfonate) (POPSO). Any derivative may be used as long as it has a pKa of about 5 to 8. The most preferred is 3-morpholinopropane- sulfonate (MOPS).

Such a buffer preferably has a pH of 5.0 to 8.0, more preferably 5.0 to 7.0, and even more preferably 5.5 to 6.5. The concentration of the buffer usually ranges from 1 to 500 mM, preferably from 5 to 100 mM, more preferably from 5 to 30 mM, and most preferably from 10 to 20 mM.

The buffer used in the present invention may be prepared in any manner. In general, a given amount of a reagent (preferably of special grade) is dissolved in purified water and adjusted to the desired pH with hydrochloric acid, sodium hydroxide, etc. Non-limiting examples of buffer preparation will be presented below.

### Acetate buffer:

Acetic acid (0.90 g) is dissolved in purified water (about 900 mL), adjusted to the desired pH with 1 M sodium hydroxide and then adjusted to a total volume of 1 L.

### Imidazole buffer:

Imidazole (1.02 g) is dissolved in purified water (about 900 mL), adjusted to the desired pH with 1 M hydrochloric acid and then adjusted to a total volume of 1 L.

### 2-Morpholinoethanesulfonate(MES) buffer:

2-Morpholinoethanesulfonate monohydrate (3.20 g) is dissolved in purified water (about 900 mL), adjusted to the desired pH with 1 M sodium hydroxide and then adjusted to a total volume of 1 L.

### 3-Morpholinopropanesulfonate (MOPS) buffer:

3-Morpholinopropanesulfonate (3.14 g) is dissolved in purified water (about 900 mL), adjusted to the desired pH with 1M sodium hydroxide and then adjusted to a total volume of 1 L.

The formulation of the present invention may further comprise a surfactant. Typical examples of a surfactant include:
nonionic surfactants (HLB 6 to 18) such as sorbitan fatty acid esters (e.g., sorbitan monocaprylate, sorbitan monolaurate, sorbitan monopalmitate), glycerine fatty acid esters (e.g., glycerine monocaprylate, glycerine monomyristate, glycerine monostearate), polyglycerine fatty acid esters (e.g., decaglyceryl monostearate, decaglyceryl distearate, decaglyceryl monolinoleate), polyoxyethylene sorbitan fatty acid esters (e.g., polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate, polyoxyethylene sorbitan tristearate), polyoxyethylene sorbitol fatty acid esters (e.g., polyoxyethylene sorbitol tetrastearate, polyoxyethylene sorbitol tetraoleate), polyoxyethylene glycerine fatty acid esters (e.g., polyoxyethylene glyceryl monostearate), polyethylene glycol fatty acid esters (e.g., polyethylene glycol distearate), polyoxyethylene alkyl ethers (e.g., polyoxyethylene lauryl ether), polyoxyethylene polyoxypropylene alkyl ethers (e.g., polyoxyethylene polyoxypropylene glycol ether, polyoxyethylene polyoxypropylene propyl ether, polyoxyethylene polyoxypropylene cetyl ether), polyoxyethylene alkylphenyl ethers (e.g., polyoxyethylene nonylphenyl ether), polyoxyethylene hydrogenated castor oils (e.g., polyoxyethylene castor oil, polyoxyethylene hydrogenated castor oil), polyoxyethylene beeswax derivatives (e.g., polyoxyethylene sorbitol beeswax), polyoxyethylene lanolin derivatives (e.g., polyoxyethylene lanolin), and polyoxyethylene fatty acid amides (e.g., polyoxyethylene stearyl amide);
anionic surfactants such as C₁₀-C₁₈ alkyl sulfates salts (e.g., sodium cetyl sulfate, sodium lauryl sulfate, sodium oleyl sulfate), polyoxyethylene C₁₀-C₁₈ alkyl ether sulfates salts with an average of 2 to 4 moles of ethylene oxide (e.g., sodium polyoxyethylene lauryl sulfate), and C₈-C₁₈ alkyl sulfosuccinate ester salts (e.g., sodium lauryl sulfosuccinate ester); and
natural surfactants such as lecithin, glycerophospholipid, sphingophospholipids (e.g., sphingomyelin), and sucrose esters of C₁₂-C₁₈ fatty acids. The formulation of the present invention may be supplemented with one or more of these surfactants.

Preferred surfactants are polyoxyethylene sorbitan fatty acid esters. Particularly preferred are Polysorbate 20, 21, 40, 60, 65, 80, 81 and 85, and most preferred are Polysorbate 20 and 80.

The formulation of the present invention may also comprise various amino acids as stabilizers. Likewise, it is further possible to use polyethylene glycol or saccharides (e.g., dextran, mannitol, sorbitol, inositol, glucose, fructose, lactose, xylose, mannose, maltose, raffinose) as an isotonizing agent.

If desired, the protein formulation of the present invention may further comprise a diluent, a solubilizer, an excipient, a pH adjuster, a soothing agent, a buffer, a sulfur-containing reducing agent, an antioxidant and the like. For instance, examples of a sulfur-containing reducing agent include those having a sulfhydryl group such as N-acetylcysteine, N-acetylhomocysteine, thioctic acid, thiodiglycol, thioethanolamine, thioglycerol, thiosorbitol, thioglycolic acid and a salt thereof, sodium thiosulfate, glutathione, and a C₁-C₇ thioalkanoic acid. Examples of an antioxidant include erythorbic acid, dibutylhydroxytoluene, butylhydroxyanisole, α-tocopherol, tocopherol acetate, L-ascorbic acid and a salt thereof, L-ascorbic acid palmitate, L-ascorbic acid stearate, sodium bisulfite, sodium sulfite, triamyl gallate and propyl gallate, as well as chelating agents such as disodium ethylenediaminetetraacetate (EDTA), sodium pyrophosphate and sodium metaphosphate. Furthermore, the protein formulation of the present invention may comprise other commonly used ingredients, for example, inorganic salts such as sodium chloride, potassium chloride, calcium chloride, sodium phosphate, potassium phosphate and sodium bicarbonate, as well as organic salts such as sodium citrate, potassium citrate and sodium acetate.

The protein formulation of the present invention is usually administered by parenteral route in the dosage form of injections (e.g., subcutaneous, intravenous, intramuscular or intraperitoneal injections) or transdermal, transmucosal, transnasal or transpulmonary preparations, but it may also be administered orally.

The protein formulation of the present invention may be a liquid formulation or a lyophilized formulation which is reconstituted before use. As excipients for lyophillization, for example, sugar alcohols or saccharides (e.g., mannitol, glucose) may be used. In the case of a liquid formulation, the protein formulation of the present invention can usually be provided in the form of containers with defined volume, including sealed and sterilized plastic or glass vials, ampoules and syringes, as well as in the form of large volume containers like bottles.

The amount of a physiologically active protein in the formulation of the present invention can be determined as appropriate for the type of physiologically active protein to be used, the type and severity of disease to be treated, the age of a patient, etc.

In general, a physiologically active protein is incorporated in an amount of 0.01 µg/ml to 200 mg/ml, preferably 0.5 µg/ml to 100 mg/ml, based on the total volume of the formulation or an injectable composition thereof, which is supplemented with saccharides. For example, in a case where a physiologically active protein is an antibody such as an immunoglobulin, a monoclonal antibody or a humanized antibody, it is usually incorporated at a final administration concentration of 0.1 to 200 mg/ml, preferably 1 to 200 mg/ml, more preferably 10 to 200 mg/ml, and most preferably 10 to 150 mg/ml. The protein formulation of the present invention shows a remarkable stabilization effect, particularly when formulated into a highly concentrated protein solution containing a physiologically active protein in an amount of 10 mg/ml or more.

The amount of a physiologically active protein in the protein-containing sample of the present invention usually ranges from 0.01 µg/ml to 200 mg/ml, preferably from 0.5 µg/ml to 100 mg/ml, based on the total volume of the sample. For example, in a case where a physiologically active protein is an antibody such as an immunoglobulin, a monoclonal antibody or a humanized antibody, it is usually incorporated at a final administration concentration of 0.1 to 200 mg/ml, preferably 1 to 200 mg/ml, more preferably 10 to 200 mg/ml, and most preferably 10 to 150 mg/ml.

Using antibody samples, the physiologically active protein formulation of the present invention was tested for the stability by fluorescence spectroscopy (Trp residue, ANS binding), cation-exchange HPLC, gel filtration HPLC and SDS-PAGE.

The results indicated that after treatment at 80°C for 2 hours, denatured intermediates (i.e., soluble aggregates) were produced in a reduced amount and hence an inhibitory effect on the denaturation phenomenon was observed in imidazole buffer, acetate buffer, 2-morpholinoethanesulfonate buffer (MES) and 3-morpholinopropanesulfonate buffer (MOPS). After treatment at 60°C for 4 weeks, on the other hand, production of thermal denatured intermediates was higher in imidazole, MES and MOPS buffers than in phosphate buffer, but no formation of insoluble aggregates was observed in these buffers. Thus, these buffers were believed to inhibit further aggregation of denatured intermediates into insoluble aggregates.

In addition, the aggregated samples were analyzed by SDS-PAGE with 2-mercaptoethanol (reducing SDS-PAGE) to give the same electrophoresis pattern as the monomer sample, thus indicating that most of the covalent linkages found in aggregates were disulfide linkages. After treatment at 60°C, however, higher-molecular-weight bands were observed even in reducing SDS-PAGE, suggesting that a part of the covalent linkages contributed to non-disulfide linking.

Meanwhile, in order to assess the effect of pH on the stability of physiologically active proteins, the thermal denaturation phenomenon and chemical changes after treatment at 60°C were assessed for both phosphate and MES buffers using antibody samples.

The thermal denaturation phenomenon was measured by cation-exchange HPLC, gel filtration HPLC and ANS-binding fluorescence spectroscopy, indicating that thermal denaturation after treatment at 60°C for 4 weeks was most effectively inhibited around pH 6. This could be explained by the results of differential scanning calorimetry. Namely, it was considered that under acidic conditions, the reduction of heat stability would be caused by low denaturation midpoint temperatures in all 3 steps of denaturation, while under basic conditions, it would be caused by the high tendency of intermediates to aggregate after denaturation.

Chemical changes were assessed by SDS-PAGE, ammonia content and the isomerization degree of aspartic acid (including asparagine), indicating that under acidic conditions, hydrolysis was very likely to occur between aspartic acid residue 272 and proline residue 273 (Asp-Pro) on the H chain, and isomerization of aspartic acid residues was also likely to occur. Under basic conditions, it was indicated that not only deamidation of asparagine and glutamine residues, but also isomerization of aspartic acid residues was likely to occur. These results confirmed that the most effective inhibition of chemical changes occurred under near-neutral conditions. In addition, hydrolysis of the Asp-Pro sequence proceeded about 10 times slower than in the corresponding peptide, suggesting that it was important to maintain the protein's higher-order structure in inhibiting chemical changes.

Further, antibody fragments (Fab and Fc fragments) were treated at 60°C and analyzed by gel filtration HPLC and ANS-binding fluorescence spectroscopy, indicating that the Fab fragment showed undetectable denaturation after heat treatment, regardless of buffer types, whereas the Fc fragment showed a buffer-dependent thermal denaturation phenomenon, as in the case of the whole antibody. Acetate and MES buffers were found to inhibit thermal denaturation of the Fc fragment, as compared to phosphate and citrate buffers. Likewise, differential scanning calorimetry was performed to assess thermal denaturation temperatures and the reversibility of thermal denaturation phenomenon, indicating that in the case of the Fab domain alone, no aggregation was observed in all buffers after denaturation. This suggested that the Fc domain would greatly contribute to aggregation of the whole antibody, and that the denaturation phenomenon of the whole antibody would be influenced by the interaction between Fab and Fc domains, rather than being equal to the mere sum of both domains.

These results indicated that the physiologically active protein formulation of the present invention would have a very excellent stabilization effect.

Thus, the present invention provides a method for improving the stability of a protein formulation, which comprises dissolving a physiologically active protein in one or more buffers selected from the group consisting of acetate buffer, imidazole buffer, a buffer of aminoethanesulfonate or a derivative thereof, and a buffer of aminopropanesulfonate or a derivative thereof.

The present invention further provides a method for stabilizing a protein-containing sample under heat treatment, which comprises performing heat treatment on the protein-containing sample in such a form that a physiologically active protein is contained in one or more buffers selected from the group consisting of acetate buffer, imidazole buffer, a buffer of aminoethanesulfonate or a derivative thereof, and a buffer of aminopropanesulfonate or a derivative thereof. The stabilizing method of the present invention is particularly advantageous for use in heat treatment for virus removal in the purification process of protein formulations, etc.

The present invention will be further described in the following Examples, which are not intended to limit the scope of the invention. Based on the detailed description, various changes and modifications will be apparent to those skilled in the art, and such changes and modifications fall within the scope of the invention.

### EXAMPLES

### Example 1: Effects of buffer type and pH on heat stability 1-1. Preparation of sample solutions

A humanized anti-IL-6 receptor antibody was used for measurement. Procedures for production of the humanized anti-IL-6 receptor antibody can be found in WO92/19759. Among antibodies found in WO92/19759, an antibody comprising the L chain version "a" and the H chain version "f" was used in the present invention. This antibody was recombinantly produced in CHO cells.

A stock solution of the humanized anti-IL-6 receptor antibody (about 40 mg/mL, pH 6.5) was diluted with 15 mM. phosphate buffer (pH 6.5) to prepare a sample solution with an antibody concentration of 10 mg/mL, which was then filled into glass ampoules (2 mL each) and flame-sealed (Sample 1: phosphate buffer, pH 6.5). pH as used herein refers to the pH of the prepared sample.

The solution with an antibody concentration of 10 mg/mL was further dialyzed against the buffers shown below to prepare sample solutions in different buffers, which were then filled into glass ampoules (2 mL each) and flame-sealed. The buffer concentration was set to 15 mM in all cases.
Sample 2: phosphate buffer, pH 5.5
Sample 3: citrate buffer, pH 6.5
Sample 4: citrate buffer, pH 5.5
Sample 5: imidazole buffer, pH 6.5
Sample 6: acetate buffer, pH 5.5
Sample 7: 2-morpholinoethanesulfonate (MES), pH 6.5
Sample 7: 2-morpholinoethanesulfonate (MES), pH 5.5
Sample 9: 3-morpholinopropanesulfonate (MOPS), pH 6.5

These sample solutions were heat-treated under conditions of 80°C for 2 hours and at 60°C for 2 or 4 weeks and then provided for stability assessment.

### 1-2. Assessment procedures

The heat-treated samples were assessed for their physical properties in the following manner to compare their stability.

### 1-2-1. Fluorescence spectroscopy (tryptophan residues)

Each sample was diluted with 15 mM phosphate buffer (pH 6.5) to give an antibody concentration of 133 µg/mL (0.89 µM) and used as a test sample. Each test sample (about 1.5 mL) was introduced into a fluorescence cell and measured for the fluorescence spectrum between 280 and 400 nm with an excitation wavelength of 280 nm. This measurement is intended to analyze the state of tryptophan residues. Upon protein denaturation, tryptophan residues in hydrophobic region will undergo structural changes to increase the fluorescence intensity and to give the maximum fluorescence wavelength shifted to a longer wavelength.
Instrument used: Spectrophotofluorometer F-2000 (Hitachi)

### 1-2-2. ANS-binding fluorescence spectroscopy

Each sample was diluted with 15 mM phosphate buffer (pH 6.5) to give an antibody concentration of 667 µg/mL (0.89 µM), 900 µL of which was supplemented with a 0.4 M solution of 1-anilino-8-naphthalene sulfonate (ANS) (300 µL) for use as a test sample. The whole volume of each test sample was introduced into a fluorescence cell and measured for the fluorescence spectrum and intensity between 450 and 550 nm with an excitation wavelength of 365 nm. ANS has the property of binding to hydrophobic regions on the protein surface and emits fluorescence upon binding. Thus, this measurement is intended to analyze the hydrophobicity of the protein surface as a change in fluorescence intensity.
Instrument used: Spectrophotofluorometer F-2000 (Hitachi)

### 1-2-3. Cation-exchange HPLC

Each sample was measured under the following analysis conditions:
Column: Resourse S 1 mL (Pharmacia)
Mobile phase: linear gradient of the following two buffers:
Solution A: 20 mM MES (pH 6.0)
Solution B: 20 mM MES (pH 6.0)/0.5 M sodium chloride

| Time (min) | A (%) | B (%) |
|---|---|---|
| 0 | 100 | 0 |
| 60 | 0 | 100 |
| 65 | 0 | 100 |
| 66 | 100 | 0 |

Flow rate: 1 mL/min
Detection wavelength: 280 nm
Injected sample amount: 10 to 30 µg (as an antibody)
HPLC device: Module-I (Waters)

### 1-2-4. Gel filtration HPLC

Each sample was measured under the following analysis conditions:
Guard column: TSK-guardcolumn SW_{XL} (Tosoh)
Separation column: TSK-gel G4000SW_{XL} (Tosoh)
Mobile phase: 50 mM phosphate buffer (pH 7.0)/0.3 M sodium chloride
Flow rate: 1 mL/min
Detection wavelength: 280 nm
Injected sample amount: 10 to 30 µg (as an antibody)
HPLC device: Module-I (Waters)

### 1-2-5. SDS-PAGE

Each sample (1 µg as an antibody) in 10% SDS-containing dilution buffer was applied to a polyacrylamide gel (12% for reducing, 8% for non-reducing) and electrophoresed for about 40 minutes with a maximum voltage of 200 V and a maximum current of 200 mA.

The bands were stained with coomassie brilliant blue.

### 2. Results

### 2-1. Fluorescence spectroscopy (Trp residues) and fluorescence spectroscopy (ANS binding)

Table 1 below shows the respective test results after heat treatment at 80°C for 2 hours.

**Table 1**

| (at 80°C for 2 hours) | | | | |
|---|---|---|---|---|
| | | | Fluorescence intensity εm=330nm (εx = 280 nm) | ANS binding εm=470nm (εx = 365 nm) |
| Sample 1 | Phosphate buffer | pH 6.5 | 5774 | 6089 |
| Sample 2 | Phosphate buffer | pH 5.5 | 5221 | 4487 |
| Sample 3 | Citrate buffer | pH 6.5 | 5779 | 6677 |
| Sample 4 | Citrate buffer | pH 5.5 | 5556 | 6511 |
| Sample 5 | Imidazole buffer | pH 6.5 | 5020 | 3003 |
| Sample 6 | Acetate buffer | pH 5.5 | 4770 | 2776 |
| Sample 7 | MES buffer | pH 6.5 | 5185 | 3665 |
| Sample 8 | MES buffer | pH 5.5 | 4670 | 1503 |
| Sample 9 | MOPS buffer | pH 6.5 | 5143 | 3306 |

All of the heat-treated samples showed increased fluorescence intensity for both spectra due to the thermal denaturation phenomenon. In a control sample (heat-untreated), the fluorescence intensity was about 4500 and the ANS binding was about 250.

When compared to phosphate and citrate buffers, imidazole, acetate, MES and MOPS buffers showed lower fluorescence intensity for both spectra after heat treatment at 80°C for 2 hours; these buffers were found to inhibit denaturation.

When compared between pH 6.5 and 5.5 for the same buffer, denaturation was inhibited more effectively at pH 5.5.

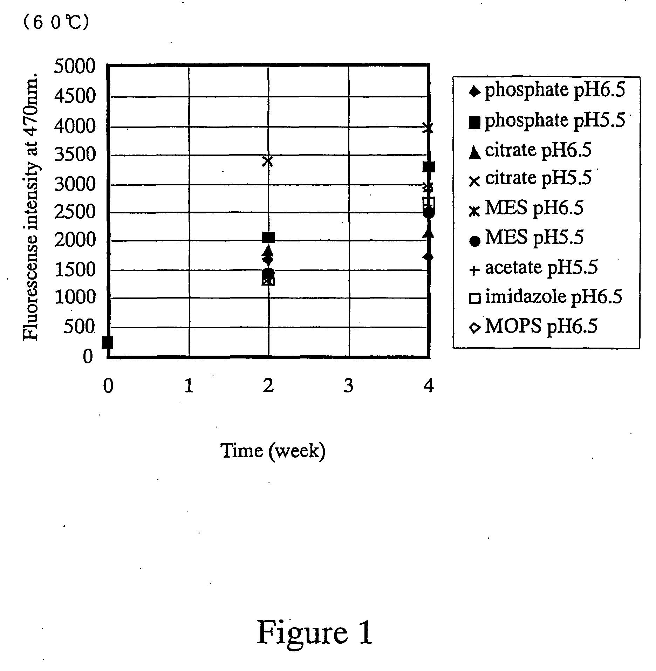

Figure 1 shows the test results of ANS binding after heat treatment at 60°C for 2 and 4 weeks.

When compared to phosphate and citrate buffers, imidazole, acetate, MES and MOPS buffers were found to inhibit denaturation. In fact, no insoluble aggregate was observed in the samples using imidazole, acetate, MES and MOPS buffers.

When compared between pH 6.5 and 5.5 for the same buffer, ANS binding was higher at pH 5.5 in contrast to the results at 80°C .

### 2-2. Cation-exchange HPLC

Table 2 below shows the test results after heat treatment at 80°C for 2 hours.

**Table 2**

| (at 80°C for 2 hours) | | | | |
|---|---|---|---|---|
| | | | Native molecule | Denatured intermediate |
| Sample 1 | Phosphate buffer | pH 6.5 | 170890 | 410576 |
| Sample 2 | Phosphate buffer | pH 5.5 | 350017 | 225267 |
| Sample 3 | Citrate buffer | pH 6.5 | 122496 | 448531 |
| Sample 4 | Citrate buffer | pH 5.5 | 144343 | 409860 |
| Sample 5 | Imidazole buffer | pH 6.5 | 383687 | 167740 |
| Sample 6 | Acetate buffer | pH 5.5 | 466018 | 160059 |
| Sample 7 | MES buffer | pH 6.5 | 351641 | 238077 |
| Sample 8 | MES buffer | pH 5.5 | 590792 | Not detected |
| Sample 9 | MOPS buffer | pH 6.5 | 380758 | 168208 |

Heat treatment at 80°C for 2 hours reduced the peak area of the native molecules, while producing a peak of denatured intermediates.

When compared to phosphate and citrate buffers, imidazole, acetate, MES and MOPS buffers showed a higher content of the native molecules remaining after treatment at 80°C for 2 hours; these buffers were found to inhibit the formation of denatured intermediates.

When compared between pH 6.5 and 5.5 for the same buffer, the content of the remaining native molecules was higher at pH 5.5, indicating that the formation of denatured intermediates was inhibited more effectively at pH 5.5.

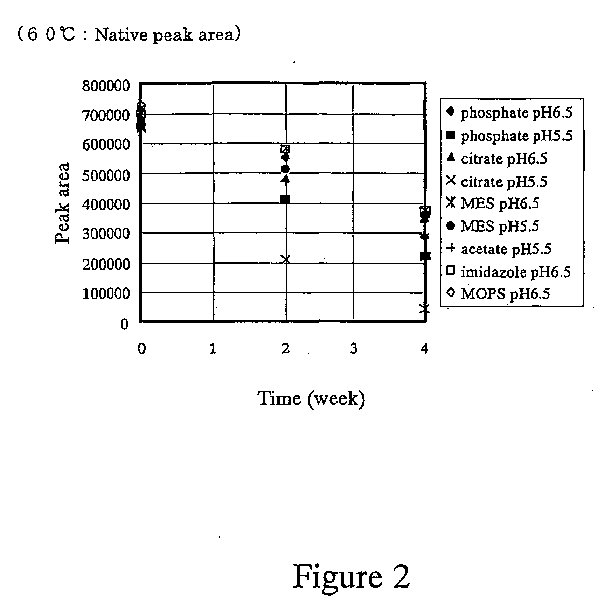

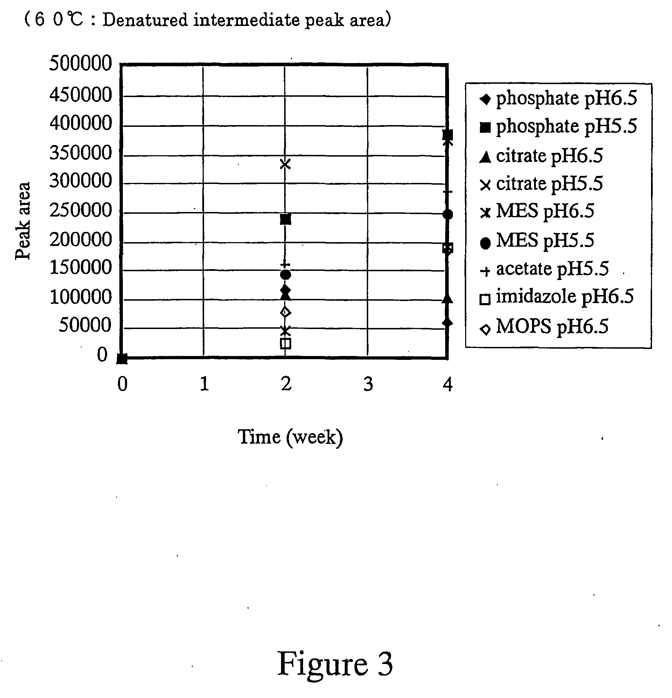

Figures 2 and 3 show the test results after treatment at 60°C for 2 and 4 weeks.

When compared to phosphate and citrate buffers, imidazole, acetate, MES and MOPS buffers showed a higher content of the remaining native molecules; these buffers were found to inhibit the formation of denatured intermediates. In fact, no insoluble aggregate was observed in the samples using imidazole, acetate, MES and MOPS buffers.

When compared between pH 6.5 and 5.5 for the same buffer, the content of the remaining native molecules was lower at the lower pH of 5.5 in contrast to the results at 80°C, thus resulting in higher production of denatured intermediates at pH 5.5.

### 2-3. Gel filtration HPLC

Table 3 below shows the test results after heat treatment at 80°C for 2 hours.

**Table 3**

| (at 80°C for 2 hours) | | | | |
|---|---|---|---|---|
| | | | Native molecule (monomer) | Denatured intermediate (associated) |
| Sample 1 | Phosphate buffer | pH 6.5 | 219350 | 528928 |
| Sample 2 | Phosphate buffer | pH 5.5 | 381133 | 379504 |
| Sample 3 | Citrate buffer | pH 6.5 | 178756 | 504121 |
| Sample 4 | Citrate buffer | pH 5.5 | 166392 | 584894 |
| Sample 5 | Imidazole buffer | pH 6.5 | 455483 | 286100 |
| Sample 6 | Acetate buffer | pH 5.5 | 481455 | 252296 |
| Sample 7 | MES buffer | pH 6.5 | 422489 | 353231 |
| Sample 8 | MES buffer | pH 5.5 | 585661 | 76946 |
| Sample 9 | MOPS buffer | pH 6.5 | 412389 | 310318 |

Heat treatment at 80°C for 2 hours reduced the peak area of monomer molecules corresponding to the native molecules, while producing a peak of soluble aggregates corresponding to denatured intermediates in the higher molecular weight region.

When compared to phosphate and citrate buffers, imidazole, acetate, MES and MOPS buffers showed a higher content of the monomer molecules remaining after treatment at 80°C for 2 hours; these buffers were found to inhibit the formation of soluble aggregates.

When compared between pH 6.5 and 5.5 for the same buffer, the formation of soluble aggregates was inhibited more effectively at pH 5.5.

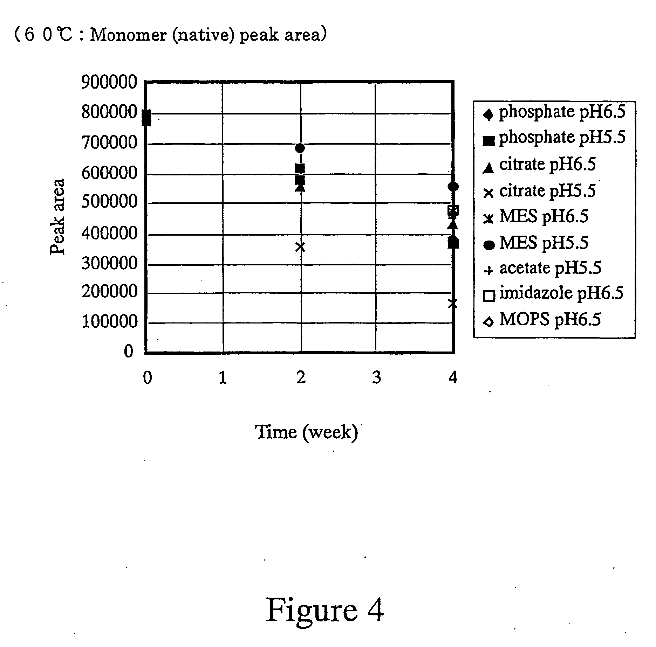

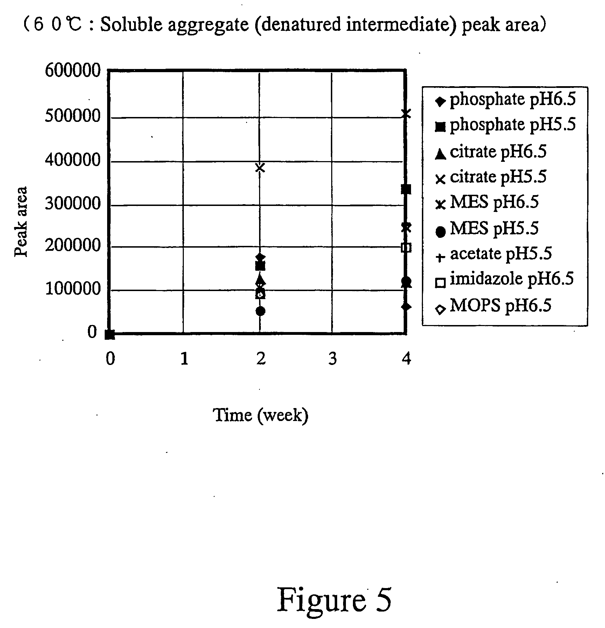

Figures 4 and 5 show the test results after treatment at 60°C for 2 and 4 weeks.

When compared to phosphate and citrate buffers, imidazole, acetate, MES and MOPS buffers showed a higher content of the remaining monomer molecules; these buffers were found to inhibit the formation of soluble aggregates. In fact, no insoluble aggregate was observed in the samples using imidazole, acetate, MES and MOPS buffers.

When compared between pH 6.5 and 5.5 for the same buffer, the content of the remaining monomer molecules was lower at the lower pH of 5.5 in contrast to the results at 80°C, thus resulting in higher production of soluble aggregates at pH 5.5.

### 2-4. SDS-PAGE

As is evident from the results of SDS-PAGE after heat treatment at 80°C for 2 hours, higher-molecular-weight bands were observed in non-reducing SDS-PAGE, which suggests the presence of covalently associated molecules. However, covalent linkages found in such associated molecules appeared to be disulfide linkages because there was no difference between treated and untreated samples in reducing SDS-PAGE.

When compared to phosphate and citrate buffers, imidazole, acetate, MES and MOPS buffers showed weaker bands in the higher molecular weight region after treatment at 80°C for 2 hours; these buffers were found to inhibit the formation of covalently associated molecules.

When compared between pH 6.5 and 5.5 for the same buffer, the formation of covalently associated molecules was inhibited more effectively at pH 5.5.

As is evident from the results of SDS-PAGE after heat treatment at 60°C for 4 weeks, when compared to phosphate and citrate buffers, imidazole, acetate, MES and MOPS buffers showed weaker bands in the higher molecular weight region; these buffers were found to inhibit the formation of covalently associated molecules. Also, detectable (but very weak) bands were observed in the higher molecular weight region even in the samples reduced with 2-mercaptoethanol (reducing SDS-PAGE), as compared to the untreated samples. This suggested that most of covalent linkages were disulfide linkages, but other chemical changes also occurred during association.

When compared between pH 6.5 and 5.5 for the same buffer, higher-molecular-weight bands in non-reducing SDS-PAGE were stronger at the lower pH of 5.5 in contrast to the results at 80°C, thus resulting in higher production of covalently associated molecules at pH 5.5.

### Example 2: Effects of pH on heat stability

### 1-1. Preparation of sample solutions

The humanized anti-IL-6 receptor antibody described in Example 1 was used for measurement. A stock solution of the humanized anti-IL-6 receptor antibody (about 40 mg/mL, pH 6.5) was dialyzed against purified water and then supplemented with buffer ingredients, followed by dilution with purified water to prepare the following sample solutions with an antibody concentration of 10 mg/mL. Each sample was filled into glass ampoules (2 mL each) and flame-sealed.
Sample 1: 15 mM phosphate buffer, pH 4
Sample 2: 15 mM phosphate buffer, pH 5
Sample 3: 15 mM phosphate buffer, pH 6
Sample 4: 15 mM phosphate buffer, pH 6.5
Sample 5: 15 mM phosphate buffer, pH 7
Sample 6: 15 mM phosphate buffer, pH 8
Sample 7: 15 mM phosphate buffer, pH 9
Sample 8: 15 mM morpholinoethanesulfonate (MES) buffer, pH 4
Sample 9: 15 mM MES buffer, pH 5
Sample 10: 15 mM MES buffer, pH 6
Sample 11: 15 mM MES buffer, pH 6.5
Sample 12: 15 mM MES buffer, pH 7
Sample 13: 15 mM MES buffer, pH 8
Sample 14: 15 mM MES buffer, pH 9

These sample solutions were heat-treated under conditions of 60°C for 4 weeks and then provided for stability assessment.

### 1-2. Assessment procedures

The heat-treated samples were assessed for their physical properties in the following manner to compare their stability.

### 1-2-1. ANS-binding fluorescence spectroscopy

Each sample was diluted with 15 mM phosphate buffer (pH 6.5) to give an antibody concentration of 667 µg/mL (0.89 µM), 900 µL of which was supplemented with a 0.4 M solution of 1-anilino-8-naphthalene sulfonate (ANS) (300 µL) for use as a test sample. The whole volume of each test sample was introduced into a fluorescence cell and measured for the fluorescence spectrum and intensity between 450 and 550 nm with an excitation wavelength of 365 nm.
Instrument used: Spectrophotofluorometer F-2000 (Hitachi)

### 1-2-2. Cation-exchange HPLC

Each sample was measured under the following analysis conditions:
Column: Resourse S 1 mL (Pharmacia)
Mobile phase: linear gradient of the following two buffers:
Solution A: 20 mM MES (pH 6.0)
Solution B: 20 mM MES (pH 6.0)/0.5 M sodium chloride

| Time (min) | A (%) | B (%) |
|---|---|---|
| 0 | 100 | 0 |
| 60 | 0 | 100 |
| 65 | 0 | 100 |
| 66 | 100 | 0 |

Flow rate: 1 mL/min
Detection wavelength: 280 nm
Injected sample amount: 10 to 30 µg (as an antibody)
HPLC device: Module-I (Waters)

### 1-2-3. Gel filtration HPLC

Each sample was measured under the following analysis conditions:
Guard column: TSK-guardcolumn SW_{XL} (Tosoh)
Separation column: TSK-gel G4000SW_{XL} (Tosoh)
Mobile phase: 50 mM phosphate buffer (pH 7.0)/0.3 M sodium chloride
Flow rate: 1 mL/min
Detection wavelength: 280 nm
Injected sample amount: 10 to 30 µg (as an antibody)
HPLC device: Module-I (Waters)

### 1-2-4. SDS-PAGE

Each sample (1 µg as an antibody) in 10% SDS-containing dilution buffer, which had been reduced with 2-mercaptoethanol, was applied to a polyacrylamide gel (12%) and electrophoresed for about 40 minutes with a maximum voltage of 200 V and a maximum current of 200 mA. The bands were stained with coomassie brilliant blue.

The stained gel was further scanned with a photoscanner and converted into an image file for use in calculation of the band density (turbidity) corresponding to the H chain. The calculation was carried out on a computer using the image analysis software Scion-Image to determine the % residual H chain (vs. initial).
Photoscanner: GT-7000U (Seiko Epson)

### 1-2-5. Ammonia content

The ammonia content was determined using an enzymatic assay kit based on the glutamate dehydrogenase reaction (F kit, ammonia, Roche Diagnostics) in accordance with the instructions attached to the kit.

### 1-2-6. Determination of isomerization degree of asparagine and aspartic acid residues

The antibody (about 1 mg) was dissolved in 6 M hydrochloric acid and hydrolyzed by heat treatment at 110°C for 4 hours under reduced pressure. During hydrolysis, asparagine and aspartic acid residues are both released in the form of aspartic acid. The hydrolyzed sample was lyophilized and then reconstituted with purified water (500 µL). This aspartic acid solution (20 µL) was mixed with an O-phthalaldehyde (OPA)/N-acetyl-L- cysteine (NAC) solution (10 µL) and, after 3 minutes, further mixed with 0.05 M acetate buffer (470 µL, pH 5.2). The resulting mixture was used as a test sample.

The OPA/NAC solution was prepared by mixing OPA (4 mg) with methanol (300 µL), 0.4 M borate buffer (250 µL, pH 9.4) and distilled water (390 µL), followed by addition of a 1 M NAC solution (60 µL, pH 5.5).

### Conditions for reversed-phase HPLC:

Column: Vydac C18 218TP54 (4.6 x 250 mm, Vydac)
Mobile phase: 4% acetonitrile/0.05 M acetate buffer (pH 5.8)
Flow rate: 0.4 mL/min
Detection wavelength: 350 nm
Injected sample volume: 20 to 500 µL
HPLC device: Pump L-7110 (Hitachi), UV absorption detector L-7400 (Hitachi)

### 1-3. Determination of thermal denaturation temperatures by differential scanning calorimetry

The thermal denaturation phenomenon of the antibody was assessed at each pH by differential scanning calorimetry (DSC). The measurement was carried out under the following conditions:
Protein concentration in each sample: 800 µg/mL
Temperature range: 40°C to 100°C
Heating rate: 0.5°C/min

The samples used in the measurement were prepared by diluting Samples 1 to 14 from Section 1-1 with the same buffers as used in the respective samples to give a concentration of 800 µg/mL.
Instrument used: Differential scanning calorimeter VP-DSC (MicroCal)

### 1-4. Amino acid sequencing of H-chain cleavage products

To analyze the H-chain fragmentation observed on SDS-PAGE, N-terminal amino acid sequencing was performed on individual fragments to identify cleavage sites.

The electrophoresed gel from SDS-PAGE was electrically transferred to a PVDF membrane and stained with coomassie brilliant blue, followed by excision of bands corresponding to H-chain cleavage products. The PVDF membrane pieces containing the bands of interest were desalted, dried and then provided for sequencing.

N-terminal amino acid sequencing was performed using the following instrument based on the Edman degradation technique under analysis conditions standard for such an instrument.
Instrument used: Amino acid sequencer 473A (Applied Biosystems)

### 1-5. Determination of H-chain cleavage rate

To analyze the H-chain fragmentation caused by hydrolysis between aspartic acid and proline residues (Asp-Pro sequence) adjacent to each other on the primary sequence, the cleavage rate under acidic conditions was measured. A 15-residue peptide containing the Asp-Pro sequence and its surrounding sequence found in this antibody was synthesized in a routine manner and also measured for the hydrolysis rate.

The antibody and peptide were cleaved under the following conditions:
Sample: humanized antibody or peptide containing Asp-Pro sequence
Peptide sequence: VDVSHEDPEVKFNWY
Buffer: 15 mM acetate buffer (pH 4)
Antibody/peptide concentration: 66.7 µM (10 mg/mL as an antibody)
Temperature and time for heat treatment: 60°C for 1, 2 and 4 weeks (antibody), 60°C for 6, 12, 24 and 36 hours (peptide)

In the case of the antibody, the **%** residual H chain (vs. initial) was determined in the same manner as shown in Section 1-2-4 and plotted against time to calculate the . H-chain cleavage rate from the negative slope of the resulting plot.

In the case of the peptide, the % residual peptide (vs. initial) was determined from the peak area of uncleaved peptide by reversed-phase HPLC and plotted against time to calculate the cleavage rate from the negative slope of the resulting plot.

### Conditions for reverses-phase HPLC:

Column: TSK-gel ODS-120T (4.6 × 250 mm, Tosoh)
Mobile phase: linear gradient of the following two buffers:
Solution A: 1% acetonitrile/0.05% hydrochloric acid
Solution B: 60% acetonitrile/0.05% hydrochloric acid

| Time (min) | A (%) | B (%) |
|---|---|---|
| 0 | 100 | 0 |
| 100 | 0 | 100 |

Flow rate: 0.6 mL/min
Detection wavelength: 350 nm
Injected sample volume: 50 µL (3.3 nmol as an uncleaved peptide)
HPLC device: Pump L-7110 (Hitachi), UV absorption detector L-7400 (Hitachi)

### 2. Results

### 2-1. Stability assessment after heat treatment at 60°C for 4 weeks

### 2-1-1. ANS-binding fluorescence spectroscopy

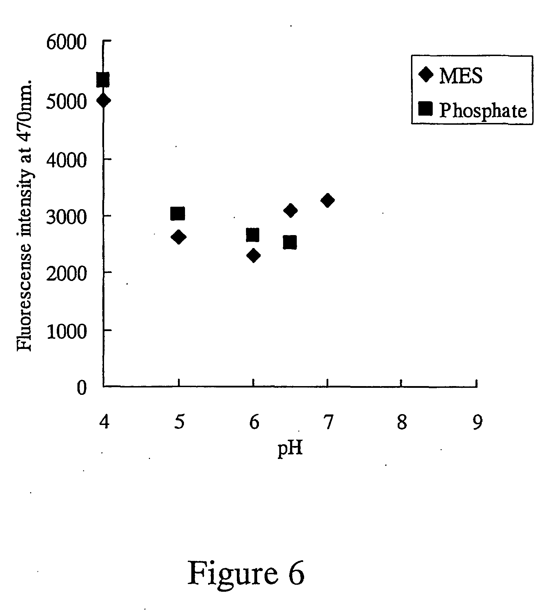

Figure 6 shows the fluorescence intensity of ANS-binding at 470 nm measured for the samples treated at 60°C for 4 weeks. The intensity was minimized at pH 6 in all buffers, indicating that the thermal denaturation phenomenon was inhibited around neutral pH. Also, the fluorescence intensity of ANS tended to be stronger under acidic conditions. In contrast, the degree of denaturation could not be assessed by ANS binding under basic conditions because denatured intermediates aggregated and precipitated.

### 2-1-2. Cation-exchange HPLC analysis

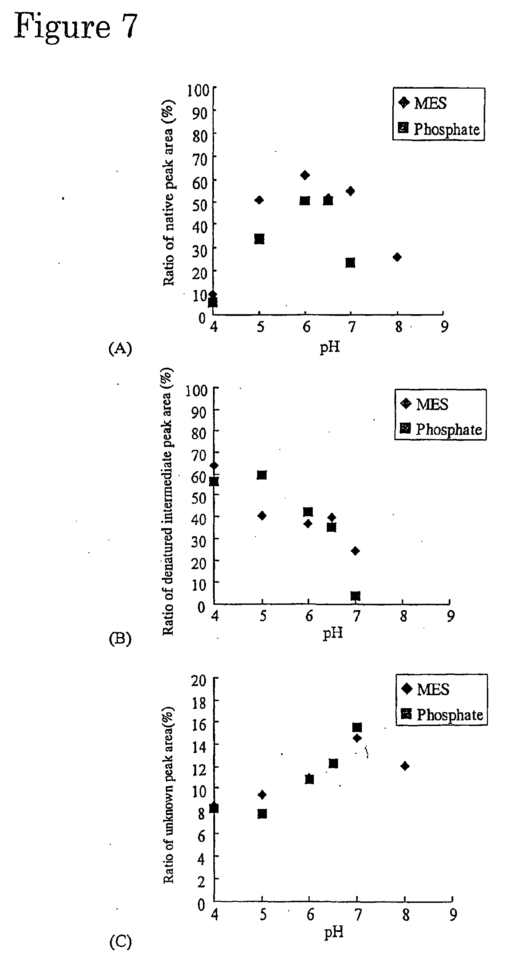

Figure 7 shows the percentages of residual native molecules and formed denatured intermediates as a result of cation-exchange HPLC analysis performed on the samples treated at 60°C for 4 weeks. In all buffers, the formation of denatured intermediates was inhibited most effectively around neutral pH. In addition, the peak area corresponding to denatured intermediates increased under acidic conditions, whereas it decreased under basic conditions. This is because under basic conditions, the apparent peak area is smaller than the real peak area since denatured intermediates appear to be unstable and further aggregated into insoluble aggregates in light of the finding that the samples produced a large amount of precipitates under basic conditions.

The unknown peak eluted faster than the native peak. reached the maximum level around neutral pH, but this unknown peak is affected by the formation of precipitates under basic conditions.

### 2-1-3. Gel filtration HPLC analysis

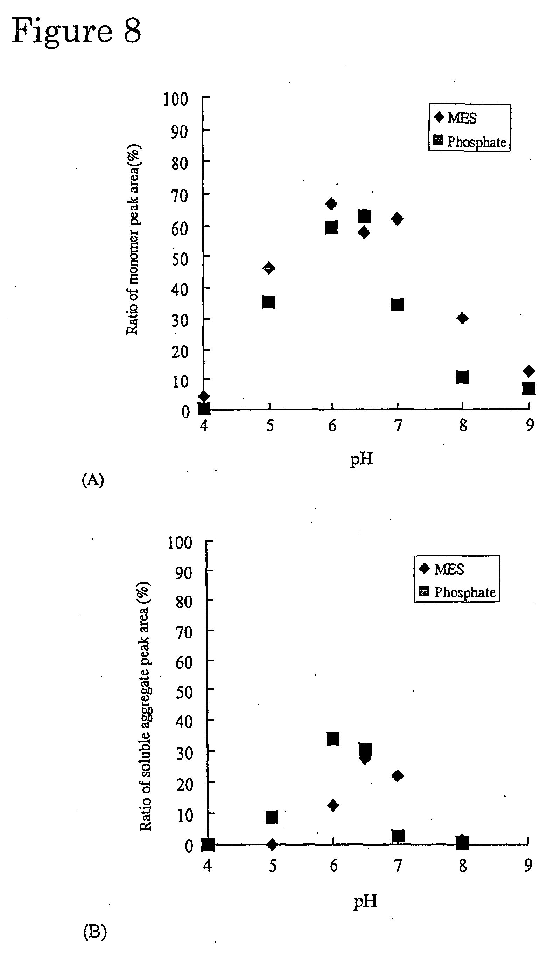

Figure 8 shows the percentages of residual monomer molecules and formed aggregates as a result of gel filtration HPLC analysis performed on the samples treated at 60°C for 4 weeks. As in the case of cation-exchange HPLC analysis, the % residual monomer molecules was the highest around neutral pH and hence the thermal denaturation phenomenon was inhibited in this pH range. Under weak acidic conditions, no formation of aggregates was observed and peaks appeared in the lower molecular weight region. Thus, these findings indicate that the peak eluted later than the native peak on cation-exchange HPLC does not result from the formation of denatured intermediates (i.e., soluble aggregates) and lower-molecular-weight cleavage products are produced instead of such aggregates.

### 2-1-4. SDS-PAGE

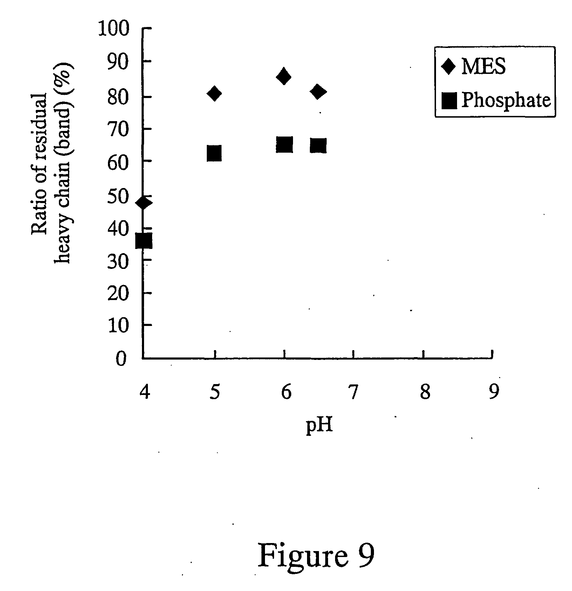

The results of reducing SDS-PAGE analysis of the samples treated at 60°C for 4 weeks indicates that the band density corresponding to the H chain was decreased under acidic conditions. In contrast, newly produced bands were observed before and after the L chain. This suggests the possibility of H chain cleavage under acidic conditions. In addition, Figure 9 shows the % residual H chain (vs. untreated H chain) assessed by quantifying the band density of the H chain. The % residual H chain was decreased at lower pH or in phosphate buffer, suggesting that H chain cleavage was facilitated.

### 2-1-5. Determination of ammonia content

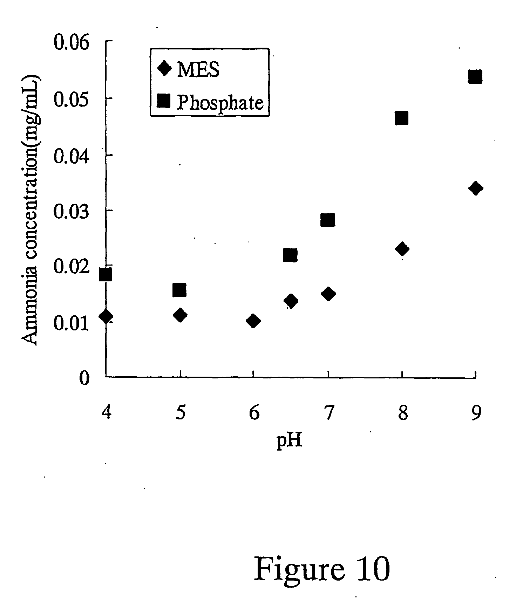

Figure 10 shows the ammonia content of the samples treated at 60°C for 4 weeks. All of the buffers were found to have a positive relationship between pH and ammonia production, in which more ammonia was produced at higher pH. Ammonia production is mostly caused by deamidation of asparagine or glutamine residues. It could therefore be confirmed that deamidation was facilitated at higher pH. Ammonia production was also higher in phosphate buffer, regardless of pH, which suggests that the probability of deamidation would vary from buffer to buffer.

Since the charge of deamidated proteins will shift to negative, deamidated antibody molecules would be contained in the above unknown peak eluted faster than the native peak on cation-exchange HPLC.

### 2-1-6. Determination of isomerization degree of asparagine and aspartic acid residues

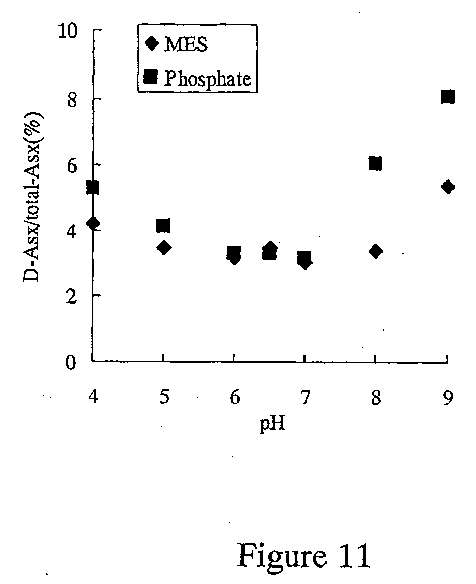

Figure 11 shows the isomerization degree of asparagine and aspartic acid residues (the ratio of D-isomers to total isomers) measured for the samples treated at 60°C for 4 weeks. The isomerization phenomenon was inhibited most effectively under neutral conditions, regardless of buffer types. In contrast, isomerization was more likely to occur under basic conditions and the isomerization degree was higher in phosphate buffer. In Figure 11, Asx denotes the sum of asparagine residues and aspartic acid residues.

### 2-2. Determination of thermal denaturation temperatures by differential scanning calorimetry

Table 4 below shows the denaturation midpoint temperatures determined by differential scanning calorimetry. All of the buffers showed a low value for each denaturation midpoint temperature at acidic pH. In contrast, an accurate determination could not be performed on the denaturation midpoint temperatures in the third step of denaturation at basic pH because aggregation was observed in this step. These results indicate that under acidic pH, destabilization would be caused by decreases in denaturation temperatures, while under basic pH, it would be caused by the high tendency of denatured molecules to aggregate. This would be responsible for high stability around pH 6.

**Table 4**

| (A) MES buffer | | | |
|---|---|---|---|
| pH | Tₘ^{1st} | Tₘ^{2nd} | Tₘ^{3rd} |
| 4 | 64.04 | 77.89 | 92.58 |
| 5 | 66.22 | 81.53 | 93.47 |
| 6 | 70.74 | 81.98 | 94.13 |
| 6.5 | 71.02 | 80.49 | 94.17 |
| 7 | 71.45 | 82.15 | 93.67* |
| 8 | 71.69 | 81.85 | 92.34* |
| 9 | 71.66 | 81.37 | 90.58* |

| | | | |
|---|---|---|---|
| *: aggregation occurred during denaturation | | | |

| (B) phosphate buffer | | | |
|---|---|---|---|
| pH | Tₘ^{1st} | Tₘ^{2nd} | Tₘ^{3rd} |
| 4 | 60.87 | 78.33 | 91.54 |
| 5 | 64.35 | 81.02 | 93.29 |
| 6 | 68.81 | 82.65 | 93.09* |
| 6.5 | 71.6 | 82.17 | 92.57* |
| 7 | 71.48 | 81.54 | 91.91* |
| 8 | 71.12 | 80.82 | 91.05* |
| 9 | 70.45 | 80.27 | 91.30* |

| | | | |
|---|---|---|---|
| *: aggregation occurred during denaturation | | | |

### 2-3. Amino acid sequencing of H-chain cleavage products

N-terminal amino acid sequencing was performed on two peaks (Unknown 1 and 2) newly found on reducing SDS-PAGE after treatment at 60°C for 4 weeks. Unknown 1 is a band of higher molecular weight than the L chain, while Unknown 2 is a band of lower molecular weight than the L chain. As a result, Unknown 1 could not be sequenced, but the sequence Pro-Glu-Val-Lys-Phe was read from the N-terminal of Unknown 2. When contrasted with the primary sequence of this antibody, this N-terminal sequence was relevant to the sequence on the H chain: - P₂₇₃-E₂₇₄-V₂₇₅-K₂₇₆-F₂₇₇- It is generally known that the aspartic acid-proline sequence is more likely to be cleaved by hydrolysis under acidic conditions, and this antibody has only one Asp-Pro sequence (D₂₇₂-P₂₇₃) over the entire sequence. Thus, fragmentation of the H chain under acidic conditions would be caused by cleavage at the "/" site in the sequence -E₂₇₁-D₂₇₂/P₂₇₃-E₂₇₄-V₂₇₅-K₂₇₆-F₂₇₇-. The reason why Unknown 1 could not be sequenced would be because the glutamine residue at the N-terminal of the H chain was pyroglutamylated and hence could not be cleaved.

### 2-4. Determination of H-chain cleavage rate

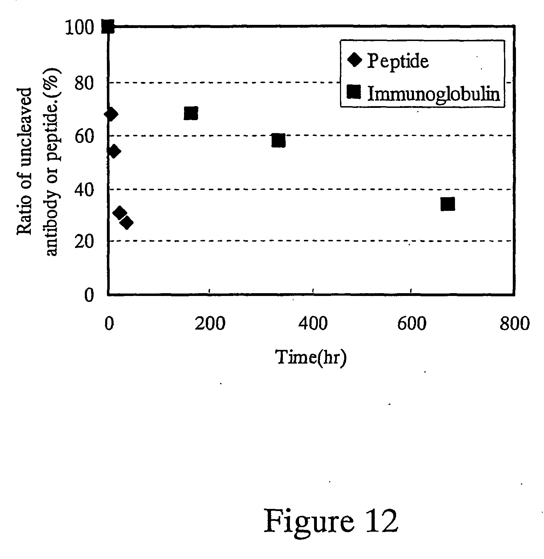

Figure 12 shows the time course of % residual uncleaved antibody and peptide during heat treatment at 60°C. As shown in this figure, a decrease in cleavage rate was observed after 1 week for the antibody H chain and after 36 hours for the peptide. Then, the initial rate of cleavage phenomena was compared between antibody and peptide, indicating that the initial rate of the antibody was about 10 times slower than that of the corresponding peptide. This is because upon exposing the Asp-Pro sequence on the antibody surface by losing the higher-order structure of antibody. Such an event constitutes a rate-limiting step of cleavage. It is therefore suggested that maintaining the antibody's native structure contributes to the inhibition of sequence cleavage between Asp and Pro.

In view of the assessment of thermal denaturation phenomenon, the maximum stability was observed around pH 6 after treatment at 60°C for 4 weeks. This can be explained by the results of differential scanning calorimetry. Namely, it is considered that under acidic conditions, destabilization would be caused by low denaturation midpoint temperatures in all 3 steps of denaturation, while under basic conditions, it would be caused by the high tendency of intermediates to aggregate after denaturation.

As to chemical changes, it was indicated that under acidic conditions, hydrolysis was very likely to occur between aspartic acid residue 272 and proline residue 273 on the H chain, and isomerization of aspartic acid residues was also likely to occur. Under basic conditions, it was indicated that not only deamidation of asparagine and glutamine residues, but also isomerization of aspartic acid residues was likely to occur. These results showed that near-neutral conditions were also most favorable in view of the assessment of chemical changes. In addition, the hydrolysis phenomenon of the Asp-Pro sequence proceeded about 10 times slower than in the corresponding peptide, suggesting that it was also important to maintain the native higher-order structure in inhibiting chemical changes.

### Example 3: Effects of buffer type on the stability of antibody fragment solutions

### 1-1. Preparation of sample solutions

### 1-1-1. Antibody fragmentation by papain digestion

The same stock solution of humanized anti-IL-6 receptor antibody (antibody concentration: about 40 mg/mL) as used in Example 1 was lyophilized and then dissolved at a concentration of 5 mg/mL in 50 mM phosphate buffer (pH 7.5, PBS) containing 0.15 M sodium chloride. This antibody solution (1 mL) was then mixed with a papain solution (1 mL) which had been dissolved at a concentration of 500 µg/mL in PBS containing 2 mM EDTA and 2 mM cysteine, followed by incubation at 37°C for 2 hours.

### 1-1-2. Purification of antibody fragments from papain digest

Fab and Fc fragments were isolated and purified from the papain digest as follows.

The papain-digested antibody fragment solution was loaded onto a Protein A column (HiTrap rProtein A FF 1 mL, Amersham Pharmacia Biotech) to collect an eluate passed through the column as a crude Fab fragment fraction. The column was washed with 0.1 M phosphate buffer (pH 7.4) containing 0.15 M sodium chloride and then eluted with 0.1 M glycine buffer (pH 3.0) to give a crude Fc fragment fraction. The crude Fc fragment fraction was neutralized with an equal volume of 0.2 M tris-hydrochloric acid buffer (pH 8.0) and then dialyzed against 0.1 M phosphate buffer (pH 7.4) containing 0.15 M sodium chloride.

The crude Fab fragment fraction was purified by cation-exchange HPLC under the following conditions:
Separation column: TSK-gel SP-5PW(Tosoh)
Mobile phase: gradient of the following two buffers:
Solution A: 50 mM acetate buffer (pH 4.0)
Solution B: 50 mM acetate buffer (pH 4.0)/0.5 M sodium chloride

| Time (min) | A (%) | B (%) |
|---|---|---|
| 0 | 100 | 0 |
| 60 | 0 | 100 |

Flow rate: 1 mL/min
Detection wavelength: 280 nm
HPLC device: Pump: L-7110 (Hitachi); UV spectrophotometric detector: L-7420 (Hitachi)

The crude Fc fragment fraction was purified by loading again the fraction onto a Protein A column. The resulting individual fragments were confirmed for their . purity by SDS-PAGE (gel concentration: 12%).

### 1-1-3. Preparation of antibody fragment solutions

Fab and Fc fragments of the humanized antibody fragmented in 1-1-1 and purified in 1-1-2 were adjusted to a concentration of 1 mg/mL and then dialyzed against the individual buffers shown below to prepare sample solutions in different buffers. Each sample solution was filled into glass ampoules (1 mL each) and flame-sealed for use in heat treatment.
Sample 1: 15 mM phosphate buffer, pH 6.5
Sample 2: 15 mM citrate buffer, pH 5.5
Sample 3: 15 mM acetate buffer, pH 5.5
Sample 4: 15 mM morpholinoethanesulfonate (MES), pH 5.5
Sample 5: 15 mM morpholinoethanesulfonate (MES), pH 6.5

Heat treatment conditions were set at 60°C for 1, 2 or 4 weeks for Fab fragment solutions and at 60°C for 2, 4 or 7 days for Fc fragment solutions. After heat treatment, these sample solutions were assessed for their stability in the following manner.

### 1-2. Assessment procedures for heat stability

The antibody fragment solutions were assessed for their heat stability by measuring a change in the peak area of monomer molecules on gel filtration HPLC and a change in the fluorescence spectrum of 1-anilino-8-naphthalene sulfonate (ANS) upon binding to a hydrophobic surface.

### 1-2-1. Gel filtration HPLC

Each sample was measured under the following analysis conditions:
Guard column: TSK-guardcolumn SWXL (Tosoh)
Separation column: TSK-gel G3000SWXL (Tosoh)
Mobile phase: 50 mM phosphate buffer (pH 7.0)/0.3 M sodium chloride
Flow rate: 1 mL/min
Detection wavelength: 280 nm
Injected sample amount: 10 to 30 µg (as an antibody fragment)
HPLC device: Pump: waters600; UV spectrophotometric detector: SPD-6A (Shimadzu)

### 1-2-2. ANS-binding fluorescence spectroscopy

The heat stability of each antibody fragment solution was measured as a change in the fluorescence spectrum of 1-anilino-8-naphthalene sulfonate (ANS) upon binding to a hydrophobic surface.

Each heat-treated or untreated antibody fragment solution (900 µL, about 10 nmol) was supplemented with a 0.4 M ANS solution (300 µL) for use as a test sample. The whole volume of each test sample was introduced into a fluorescence cell and measured for the fluorescence spectrum between 450 and 550 nm with an excitation wavelength of 365 nm.
Instrument used: Spectrophotofluorometer F-2000 (Hitachi)

### 1-3. Assessment of thermal denaturation temperatures by differential scanning calorimetry

The thermal denaturation phenomenon of Fab and Fc fragments and the whole antibody was assessed in each buffer by differential scanning calorimetry (DSC). The measurement was carried out under the following conditions:
Protein concentration in each sample: 1 mg/mL
Temperature range: 40°C to 100°C
Heating rate: 0.5°C/min

Among the test samples, the Fab and Fc fragment samples were prepared as described in 1-1. The antibody (whole) samples were prepared from the antibody stock solution by dialysis against and dilution in each buffer to give a concentration of 1 mg/mL.
Instrument used: Differential scanning calorimeter VP-DSC (MicroCal)

### 1-4. Assessment of reversibility of thermal denaturation phenomenon by differential scanning calorimetry

The Fab and Fc fragments and the whole antibody in MES buffer (15 mM, pH 5.5) were assessed for reversibility of their thermal denaturation phenomenon in each denaturation step by differential scanning calorimetry (DSC). More specifically, the Fab and Fc fragments and the whole antibody were heated to their respective denaturation midpoint temperatures in each denaturation step where the reversibility was assessed (first temperature scan). After cooling to the initial temperature, each sample was heated again (second temperature scan) to compare the height of the endothermic peak between first and second scans in each denaturation step. The measurement was carried out under the following conditions:
Protein concentration in each sample: 0.25 mg/mL
First temperature scan: 40°C to each denaturation midpoint temperature at a heating rate of 0.5°C/min
Second temperature scan: 40°C to 100°C at a heating rate of 0.5°C/min.
Instrument used: Differential scanning calorimeter VP-DSC (MicroCal)

The Fab fragment and the whole antibody in MES buffer (15 mM, pH 5.5) were also assessed for the effect of DSC heating rate on their denaturation midpoint temperature in each denaturation step. The measurement was carried out under the following conditions:
Protein concentration in each sample: 1 mg/mL
Temperature range: 40°C to 100°C
Heating rate: 0.25, 0.5, 1.0, 1.5°C/min
Instrument used: Differential scanning calorimeter VP-DSC (MicroCal)

### 2. Results

### 2-1. Preparation of antibody fragments by papain digestion

Since the results of SDS-PAGE confirmed that the Fab and Fc fragments were each highly purified, these fragments were dialyzed against each buffer and then provided for heat stability assessment.

### 2-2. Stability assessment after heat treatment

### 2-2-1. Assessment by gel filtration HPLC

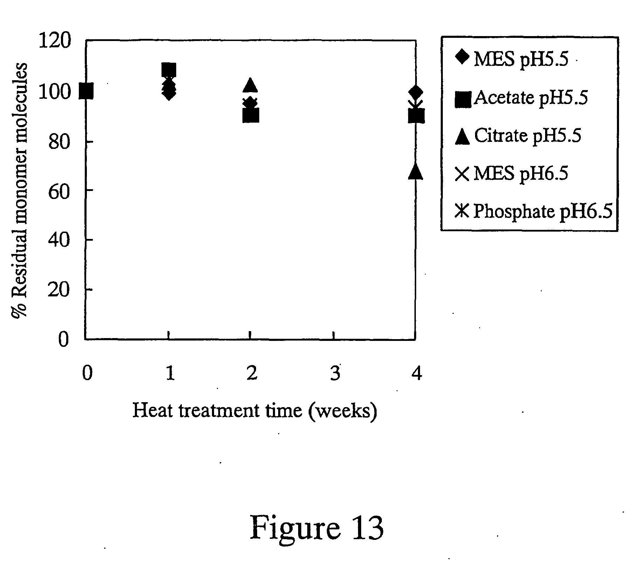

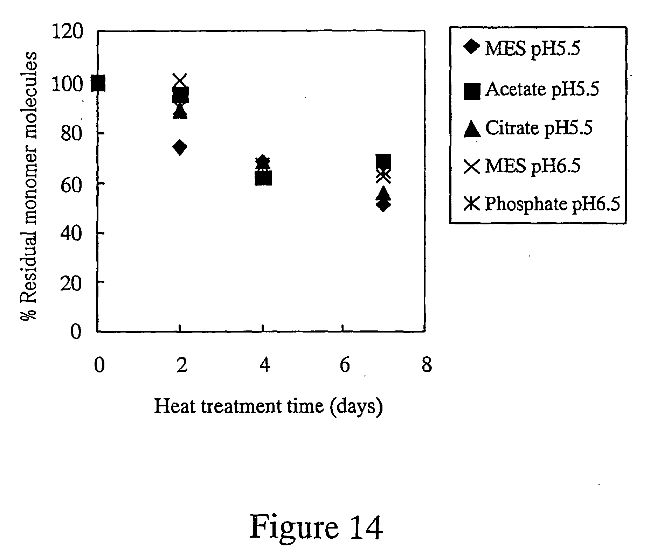

Figures 13 and 14 show the results of gel filtration HPLC analysis performed on the heat-treated samples of Fab and Fc fragments (Figure 13: Fab fragment; Figure 14: Fc fragment), which were used to determine the % residual monomer molecules.

The Fab fragment was very stable in all buffers excluding citrate buffer. The % residual monomer molecules was 90% or more in these buffers even after heat treatment at 60°C for 4 weeks, whereas it was as low as about 70% in citrate buffer.

In the Fc fragment, on the other hand, the **%** residual monomer molecules was reduced to 50%-70% after heat treatment at 60°C for 1 week, but no significant tendency was found in the difference of stability between buffer species.

### 2-2-2. Assessment by ANS-binding fluorescence spectroscopy

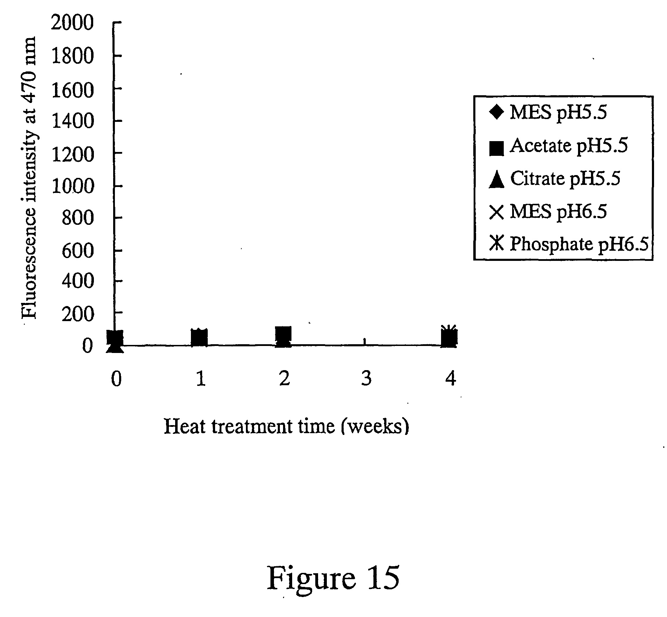

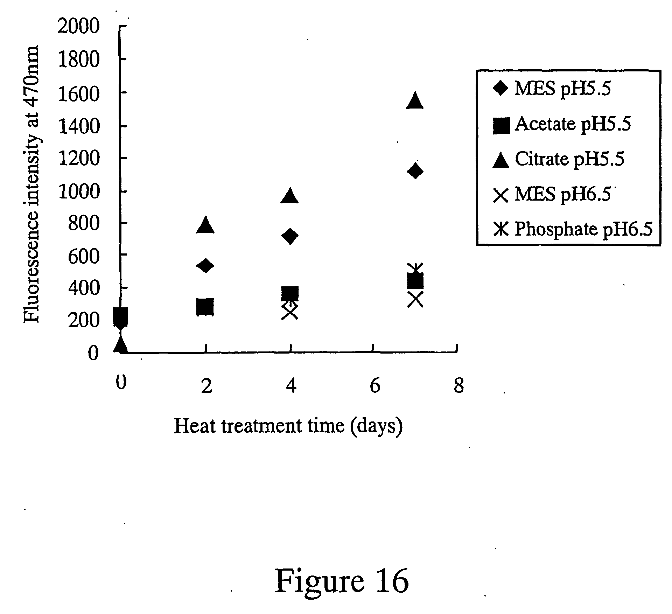

Figures 15 and 16 show the results of ANS-binding fluorescence spectroscopy performed on the heat-treated samples of Fab and Fc fragments (Figure 15: Fab fragment; Figure 16: Fc fragment), which were used to analyze the surface exposure of hydrophobic residues.

The Fab fragment showed no change in fluorescence intensity after heat treatment at 60°C for 4 weeks in all buffers; there was no binding of ANS onto the hydrophobic surface.

In contrast, the Fc fragment showed an increase in ANS-binding fluorescence intensity after heat treatment at 60°C for 1 week (7 days). When compared at the same pH, the fluorescence intensity after heat treatment was higher in phosphate and citrate buffers in which the whole antibody was less heat-stable.

Thus, the Fab fragment showed undetectable denaturation after heat treatment, regardless of buffer types, whereas the Fc fragment showed a buffer-dependent thermal denaturation phenomenon, as in the case of the whole antibody. Acetate and MES buffers were found to inhibit thermal denaturation of the Fc fragment, as compared to phosphate and citrate buffers.

### 2-3. Assessment of thermal denaturation phenomenon by differential scanning calorimetry

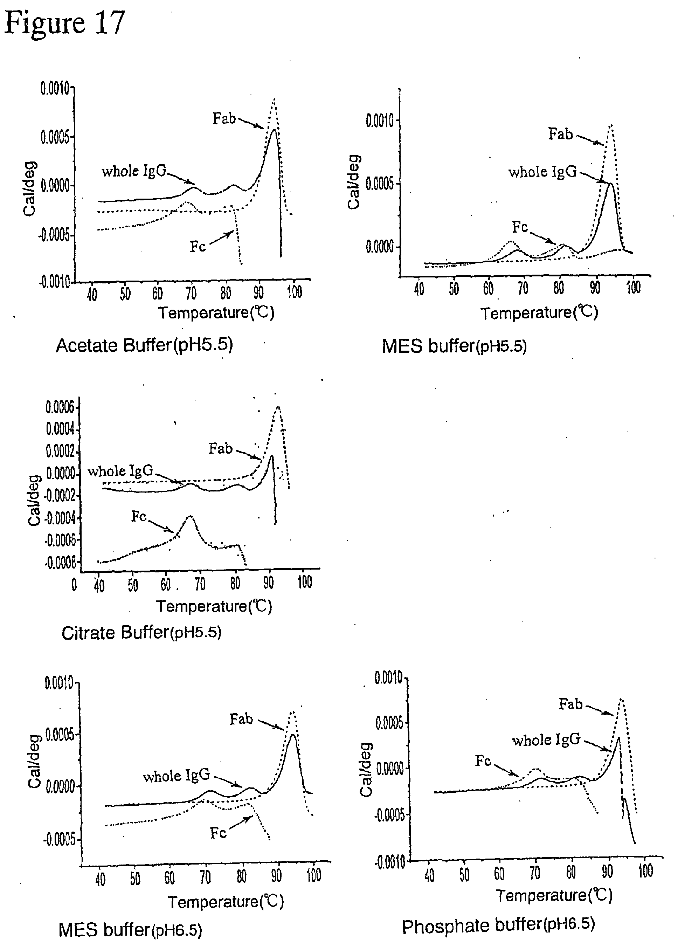

As shown in Figure 17, the whole antibody resulted in 3 endothermic peaks, the first two of which could be assigned to Fc domain denaturation, and the last of which could be assigned to Fab domain denaturation.

Except for MES buffer (pH 5.5), the whole antibody produced an exothermic peak due to aggregation after the third step of denaturation, whereas the Fab fragment showed no such a phenomenon after the corresponding denaturation. The Fc fragment also produced an exothermic peak in these buffers after the second step of denaturation. In citrate buffer (pH 5.5) and phosphate buffer (pH 6.5), such an aggregation tendency was observed not only in the Fc fragment but also in the Fab fragment. In MES buffer (pH 5.5), on the other hand, both of the whole antibody and the Fc fragment produced no exothermic peak, thus resulting in inhibition of aggregation of denatured molecule. However, there was no great difference in denaturation temperatures (Tm) between buffers, as shown in Table 5 below.

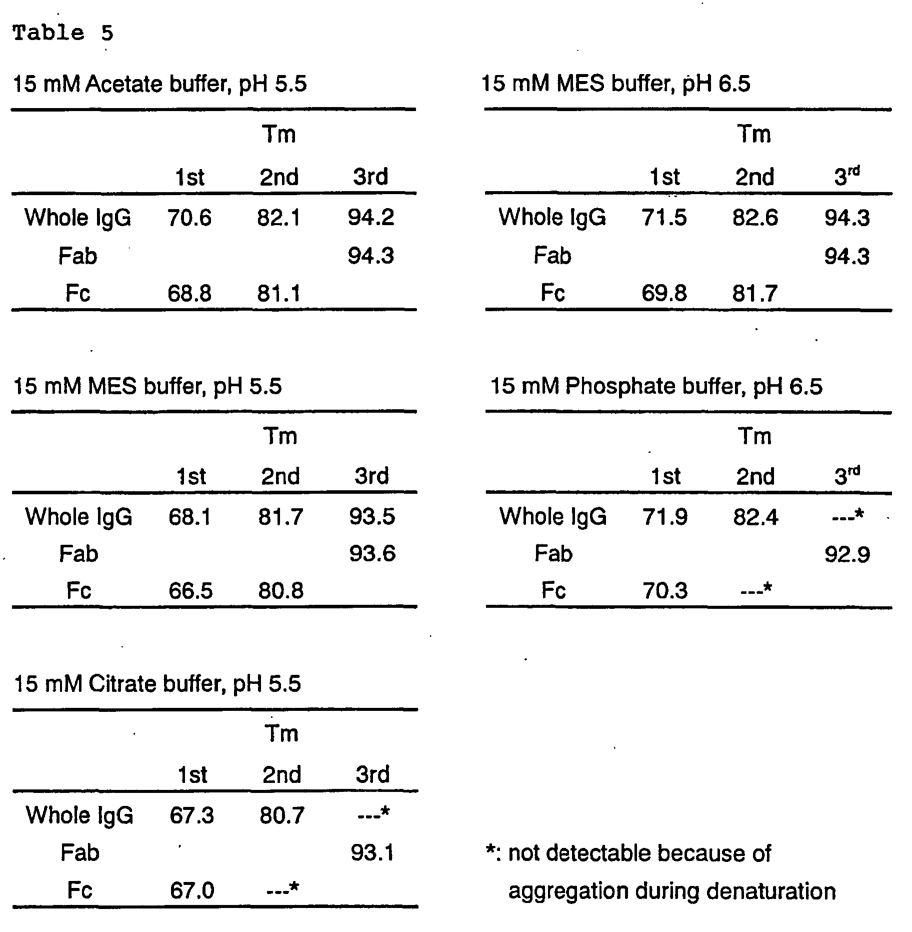

### 2-4. Assessment of reversibility of thermal denaturation phenomenon by differential scanning calorimetry

In 15 mM MES buffer (pH 5.5), the Fab and Fc fragments and the whole antibody experienced no aggregation phenomenon after thermal denaturation. Then, the thermal denaturation phenomenon in MES buffer was assessed for reversibility in each denaturation step.

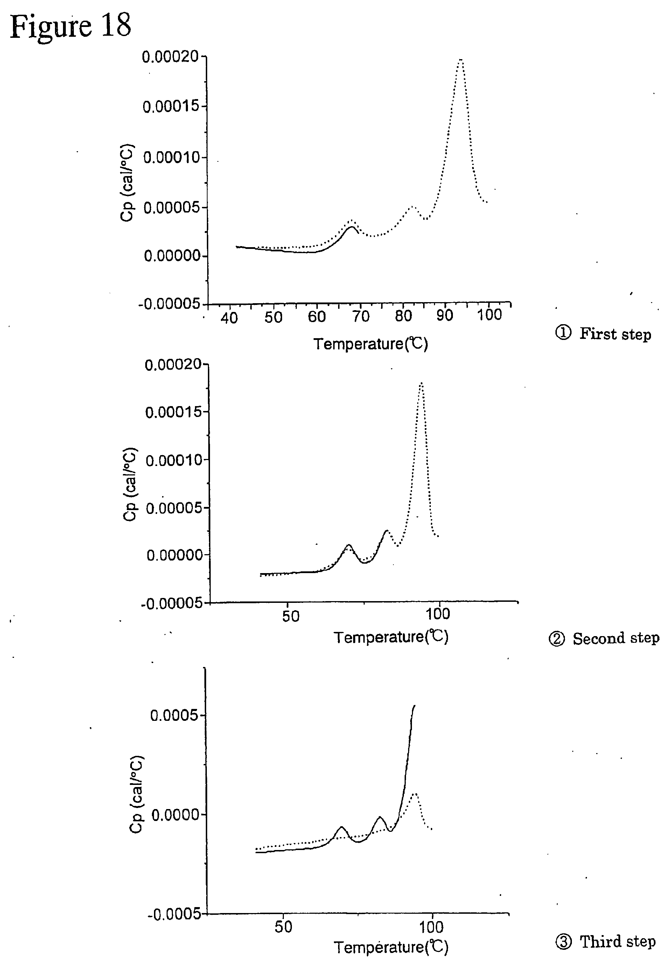

The denaturation of the whole antibody consists of three steps in total, i.e., the first two steps resulting from the Fc domain and the last step resulting from the Fab domain. The reversibility of denaturation could be observed in the first and second steps, whereas denaturation in the last third step was less reversible (Figure 18).

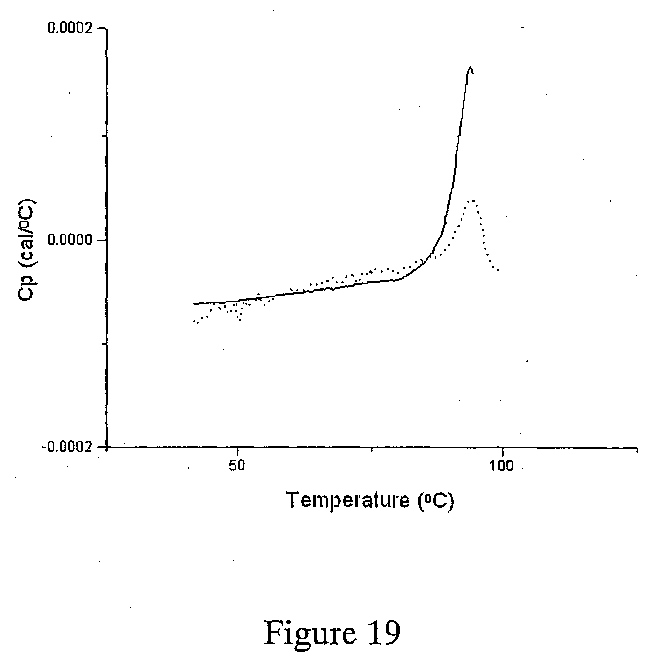

The denaturation of the Fab fragment was also less reversible, as in the case of the third denaturation step of the whole antibody where the Fab domain was denatured. However, the second temperature scan resulted in an endothermic peak higher than that of the whole antibody (Figure 19). This means that the reversibility of denaturation is higher in the Fab fragment alone than in the whole antibody, which in turn suggests that reversibility in the third denaturation step of the whole antibody would be influenced by the presence of the Fc domain.

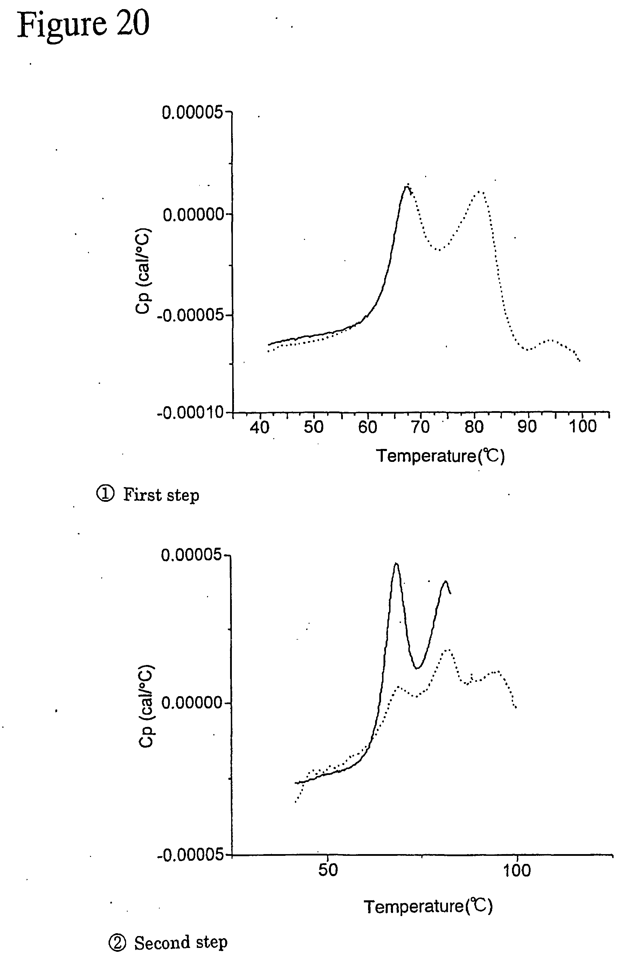

In the Fc fragment, denaturation was completely reversible in the first step, but less reversible in the second step (Figure 20). This is in contrast to the corresponding results of the Fc domain in the whole antibody and suggests that reversibility in the second denaturation step of the whole antibody would be influenced by the presence of the Fab domain.

It was therefore demonstrated that the denaturation of each antibody domain in MES buffer was irreversible, except for the first and second steps of the whole antibody and the first step of the Fc domain.

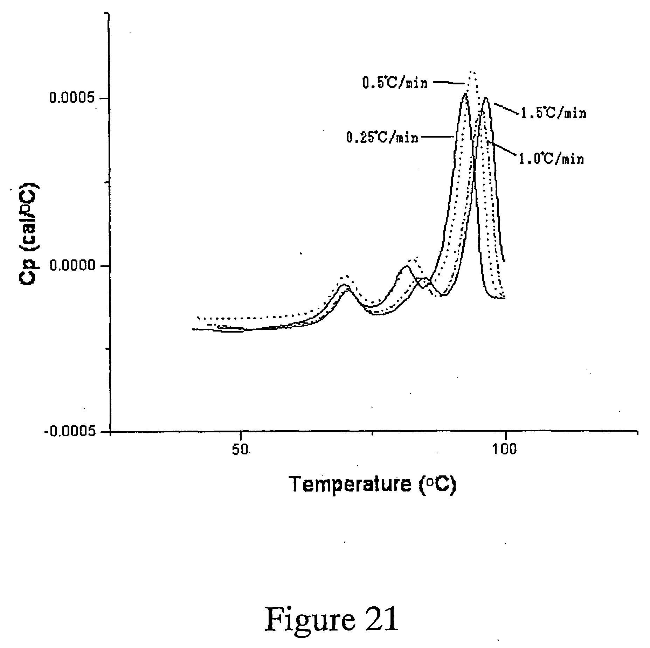

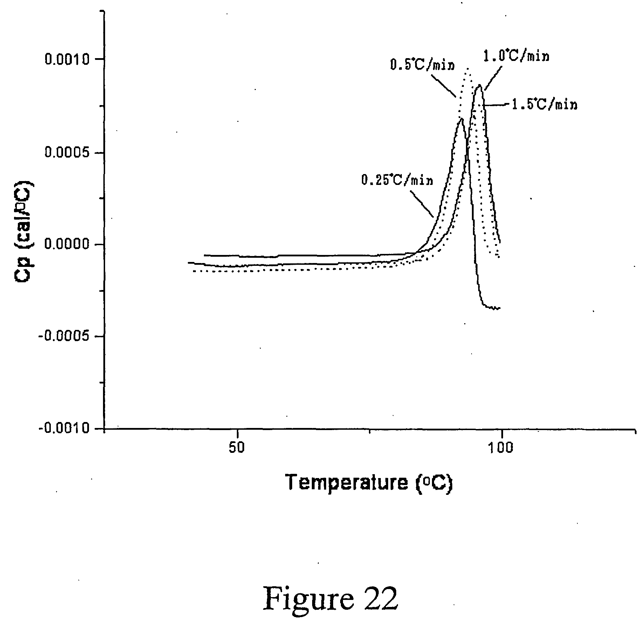

The whole antibody and the Fab fragment were also assessed for the effect of DSC heating rate on their denaturation midpoint temperatures in MES buffer. In the whole antibody, denaturation in the second and third steps resulted in a higher denaturation midpoint temperature at a higher heating rate, whereas the denaturation temperature of the first step remained constant independently of heating rate (Figure 21). Likewise, the denaturation of the Fab fragment also resulted in a higher denaturation midpoint temperature at a higher heating rate (Figure 22). These results also supported the reversibility of denaturation in the first step of the whole antibody. When the individual denaturation midpoint temperatures were plotted against heating rate and extrapolated to calculate the denaturation midpoint temperature at a heating rate of 0°C/min, the resulting value was 69.02°C, 79.83°C and 90.96°C in the whole antibody and 91.01°C in the Fab fragment.

These results indicated that the heat stabilization effect in MES buffer at pH 5.5 was ascribed to the inhibition of denatured molecule aggregation rather than increases in thermal denaturation temperatures. Namely, this suggested that the difference in stability between buffer species was ascribed to the difference in aggregation properties after denaturation rather than the difference in denaturation temperatures.

Since in the case of the Fab domain alone, no aggregation was observed in all buffers after denaturation, it was indicated that the Fc domain would greatly contribute to aggregation of the whole antibody. It was also believed that the denaturation phenomenon of the whole antibody would be influenced by the interaction between both Fab and Fc domains, rather than being equal to the mere sum of both domains.

When the reversibility of denaturation was assessed, it could be observed only in the first and second steps of the whole antibody and in the first step of the Fc domain. This suggested that the reversibility of denaturation phenomenon would make little contribution to the stability of antibody solutions.

## Claims

1. A protein formulation with improved stability, which comprises a physiologically active protein in one or more buffers selected from the group consisting of acetate buffer, imidazole buffer, a buffer of aminoethanesulfonate or a derivative thereof, and a buffer of aminopropanesulfonate or a derivative thereof.

2. The protein formulation according to claim 1, wherein the buffer(s) has a pH of 5 to 7.

3. The protein formulation according to claim 1 or 2, wherein said aminoethanesulfonate or a derivative thereof is selected from the group consisting of:
2-morpholinoethanesulfonate (MES),
N-(2-acetamide)-2-aminoethanesulfonate (ACES),
N,N-bis(2-hydroxyethyl)-2-aminoethanesulfonate (BES),
2-[4-(2-hydroxyethyl)-1-piperazinyl]ethanesulfonate (HEPES),
piperazine-1,4-bis(2-ethanesulfonate) (PIPES), and
N-tris(hydroxymethyl)methyl-2-aminoethanesulfonate (TES).

4. The protein formulation according to claim 1 or 2, wherein said aminopropanesulfonate or a derivative thereof is selected from the group consisting of:
3-morpholinopropanesulfonate (MOPS),
2-hydroxy-3-[4-(2-hydroxyethyl)-1-piperazinyl]propanesulfonate (HEPPSO),
2-hydroxy-3-morpholinopropanesulfonate (MOPSO), and
piperazine-1,4-bis(2-hydroxy-3-propanesulfonate) (POPSO).

5. The protein formulation according to claim 1 or 2, wherein the protein is selected from an antibody, an enzyme, a cytokine and a hormone.

6. The protein formulation according to claim 5, wherein the protein is an antibody.

7. The protein formulation according to claim 6, wherein the antibody is a monoclonal antibody.

8. The protein formulation according to claim 7, wherein the monoclonal antibody is a humanized antibody.

9. A method for improving the stability of a protein formulation, which comprises dissolving a physiologically active protein in one or more buffers selected from the group consisting of acetate buffer, imidazole buffer, a buffer of aminoethanesulfonate or a derivative thereof, and a buffer of aminopropanesulfonate or a derivative thereof.

10. A method for stabilizing a protein-containing sample under heat treatment, which comprises performing heat treatment on the protein-containing sample in such a form that a physiologically active protein is contained in one or more buffers selected from the group consisting of acetate buffer, imidazole buffer, a buffer of aminoethanesulfonate or a derivative thereof, and a buffer of aminopropanesulfonate or a derivative thereof.

11. A method for stabilizing the Fc region of an antibody, which comprises dissolving the antibody in one or more buffers selected from the group consisting of acetate buffer, imidazole buffer, a buffer of aminoethanesulfonate or a derivative thereof, and a buffer of aminopropanesulfonate or a derivative thereof.
